# EUROPEAN PATENT SPECIFICATION

(11) **EP 2 675 807 B1**
(45) Date of publication and mention of the grant of the patent: **22.04.2015**
(21) Application number: 12705246.2
(22) Date of filing: 13.02.2012
(51) Int. Cl.: C07D 487/04, A61K 31/519, A61P 25/28

(54) **6-CYCLOBUTYL-1, 5-DIHYDRO-PYRAZOLO [3, 4-D] PYRIMIDIN-4-ONE DERIVATIVES AND THEIR USE AS PDE9A INHIBITORS**
6-CYCLOBUTYL-1, 5-DIHYDRO-PYRAZOLO [3, 4-D] PYRIMIDIN-4-ONE UND IHRE VERWENDUNG ALS PDE9 INHIBITOREN
DÉRIVÉS DE 6-CYCLOBUTYL-1,5-DIHYDRO-PYRAZOLO[3,4-D]PYRIMIDIN-4-ONE ET LEUR UTILISATION EN TANT QU'INHIBITEURS DE PDE9A

(30) Priority: 14.02.2011 EP 11154397; 09.08.2011 WO PCT/EP2011/063705
(43) Date of publication of application: 25.12.2013
(73) Proprietor: Boehringer Ingelheim International GmbH, 55216 Ingelheim am Rhein (DE)
(72) Inventor: HEINE, Niklas, 55216 Ingelheim am Rhein (DE); GIOVANNINI, Riccardo, 55216 Ingelheim am Rhein (DE); FERRARA, Marco, 55216 Ingelheim am Rhein (DE)
(74) Representative: Simon, Elke Anna Maria
(86) International application number: PCT/EP2012/052378
(87) International publication number: WO 2012/110440

(56) References cited:
- WO-A1-2004/099211
- WO-A1-2009/121919
- WO-A1-2010/026214
- WO-A1-2011/018495

## Description

The invention relates to novel pyrazolopyrimidinones according to formula (I) 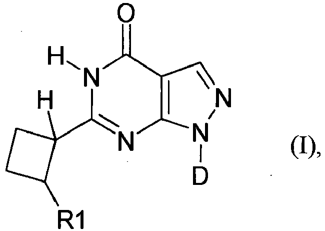 wherein R¹ is a pyridyl or pyrimidinyl group and D is optionally substituted cyclopentyl, cyclohexyl, tetrahydrofuranyl, tetrahydropyranyl or 2-, 3- or 4-pyridyl.

The new compounds are for use as the active entity of medicaments or for the manufacture of medicaments respectively, in particular medicaments for the treatment of conditions concerning deficits in perception, concentration, learning or memory. Such conditions may for example be associated with Alzheimer's disease, schizophrenia and other diseases. The new compounds are also for example for the manufacture of medicaments and/or for use in the treatment of these diseases, in particular for cognitive impairment associated with such disease. The compounds of the invention show PDE9 inhibiting properties.

### BACKGROUND OF THE INVENTION

The inhibition of phosphodiesterase 9A (PDE9A) is one of the current concepts to find new access paths to the treatment of cognitive impairments due to CNS disorders like Alzheimer's disease, schizophrenia and other diseases or due to any other neurodegenerative process of the brain. With the present invention, new compounds that follow this concept are presented. Phosphodiesterase 9A is one member of the wide family of phosphodiesterases. These enzymes modulate the levels of the cyclic nucleotides 5'-3' cyclic adenosine monophosphate (cAMP) and 5'-3' cyclic guanosine monophosphate (cGMP). These cyclic nucleotides (cAMP and cGMP) are important second messengers and therefore play a central role in cellular signal transduction cascades. Each of them reactivates inter alia, but not exclusively, protein kinases. The protein kinase activated by cAMP is called protein kinase A (PKA) and the protein kinase activated by cGMP is called protein kinase G (PKG). Activated PKA and PKG are able in turn to phosphorylate a number of cellular effector proteins (e.g. ion channels, G-protein-coupled receptors, structural proteins, transcription factors). It is possible in this way for the second messengers cAMP and cGMP to control a wide variety of physiological processes in a wide variety of organs. However, the cyclic nucleotides are also able to act directly on effector molecules. Thus, it is known, for example, that cGMP is able to act directly on ion channels and thus is able to influence the cellular ion concentration (review in: Wei et al., Prog. Neurobiol., 1998, 56, 37-64). The phosphodiesterases (PDE) are a control mechanism for the activity of cAMP and cGMP and thus in turn for the corresponding physiological processes. PDEs hydrolyse the cyclic monophosphates to the inactive monophosphates AMP and GMP. Currently, 11 PDE families have been defined on the basis of the sequence homology of the corresponding genes. Individual PDE genes within a family are differentiated by letters (e.g. PDE1A and PDE1B). If different splice variants within a gene also occur, then this is indicated by an additional numbering after the letters (e.g. PDE1A1).

Human PDE9A was cloned and sequenced in 1998. The amino acid identity with other PDEs does not exceed 34% (PDE8A) and is never less than 28% (PDE5A). With a Michaelis-Menten constant (Km) of 170 nanomolar (nM), PDE9A has high affinity for cGMP. In addition, PDE9A is selective for cGMP (Km for cAMP=230 micromolar (µM)). PDE9A has no cGMP binding domain, suggesting that the enzyme activity is not regulated by cGMP. It was shown in a Western blot analysis that PDE9A is expressed in humans inter alia in testes, brain, small intestine, skeletal muscle, heart, lung, thymus and spleen. The highest expression was found in the brain, small intestine, kidney, prostate, colon and spleen (Fisher et al., J. Biol. Chem., 1998, 273 (25), 15559-15564; Wang et al., Gene, 2003, 314, 15-27). The gene for human PDE9A is located on chromosome 21q22.3 and comprises 21 exons. 4 alternative splice variants of PDE9A have been identified (Guipponi et al., Hum. Genet., 1998, 103, 386-392). Classical PDE inhibitors do not inhibit human PDE9A. Thus, IBMX, dipyridamole, SKF94120, rolipram and vinpocetine show no inhibition on the isolated enzyme in concentrations of up to 100 micromolar (µM). An IC50 of 35 micromolar (µM) has been demonstrated for zaprinast (Fisher et al., J. Biol. Chem., 1998, 273 (25), 15559-15564).

Murine PDE9A was cloned and sequenced in 1998 by Soderling et al. (J. Biol. Chem., 1998, 273 (19), 15553-15558). This has, like the human form, high affinity for cGMP with a Km of 70 nanomolar (nM). Particularly high expression was found in the mouse kidney, brain, lung and liver. Murine PDE9A is not inhibited by IBMX in concentrations below 200 micromolar either; the IC50 for zaprinast is 29 micromolar (Soderling et al., J. Biol. Chem., 1998, 273 (19), 15553-15558). It has been found that PDE9A is strongly expressed in some regions of the rat brain. These include olfactory bulb, hippocampus, cortex, basal ganglia and basal forebrain (Andreeva et al., J. Neurosci., 2001, 21 (22), 9068-9076). The hippocampus, cortex and basal forebrain in particular play an important role in learning and memory processes. As already mentioned above, PDE9A is distinguished by having particularly high affinity for cGMP. PDE9A is therefore active even at low physiological concentrations, in contrast to PDE2A (Km=10 micromolar (µM); Martins et al., J. Biol. Chem., 1982, 257, 1973-1979), PDE5A (Km=4 micromolar (µM); Francis et al., J. Biol. Chem., 1980, 255, 620-626), PDE6A (Km=17 micromolar (µM); Gillespie and Beavo, J. Biol. Chem., 1988, 263 (17), 8133-8141) and PDE11A (Km=0.52 micromolar (µM); Fawcett et al., Proc. Nat. Acad. Sci., 2000, 97 (7), 3702-3707). In contrast to PDE2A (Murashima et al., Biochemistry, 1990, 29, 5285-5292), the catalytic activity of PDE9A is not increased by cGMP because it has no GAF domain (cGMP-binding domain via which the PDE activity is allosterically increased) (Beavo et al., Current Opinion in Cell Biology, 2000, 12, 174-179). PDE9A inhibitors may therefore lead to an increase in the baseline cGMP concentration.

This outline will make it evident that PDE9A engages into specific physiological processes in a characteristic and unique manner, which distinguishes the role of PDE9A characteristically from any of the other PDE family members:
WO 2004/099210 discloses 6-arylmethyl-substituted pyrazolopyrimidinones which are PDE9 inhibitors.
WO 2004/099211 discloses 6-cyclylmethyl- and 6-alkylmethyl-substituted pyrazolopyrimidines and their use for the improvement of cognition, concentration etc..
DE 102 38 722 discloses the use of PDE9A-inhibitors for the improvement of cognition, concentration.
WO 2004/018474 discloses phenyl-substituted pyrazolopyrimidines and their use for the improvement of perception, concentration learning and/or memory.
WO 2004/026876 discloses alkyl-substituted pyrazolopyrimidines which and their use for the improvement of awareness, concentration learning capacity and/or memory performance.
WO 2004/096811 discloses heterocyclic bicycles as PDE9 inhibitors for the treatment of diabetes, including type 1 and type 2 diabetes, hyperglycemia, dyslipidemia, impaired glucose tolerance, metabolic syndrome and/or cardiovascular disease.
WO2009068617 discloses PDE9 inhibiting compounds derived from pyrazolopyrimidinones with a substituted phenylmethyl- or pyridyl-methyl group in the 6-position.
WO2010112437 discloses PDE9 inhibiting compounds derived from pyrazolopyrimidinones with a phenyl or heteroaryl substituted arylmethyl- or heteroaryl-methyl group in the 6-position.
WO 2009/121919 discloses PDE9 inhibitors derived from pyrazolopyrimidinones with a nonaromatic heterocyclyl group in the 1-position, among which is tetrahydropyranyl.
WO 2010/026214 discloses PDE9 inhibitors derived from pyrazolopyrimidinones with a cycloalkyl or a cycloalkenyl group in the 1-position, among which is 4,4-difluorocyclohexyl.

Some prior art is directed to chemically nucleoside derivatives. As examples it is referred to WO 2002/057425, which discloses nucleoside derivatives, which are inhibitors of RNA-dependent RNA viral polymerase, or WO 2001/060315, which discloses nucleoside derivatives for the treatment of hepatitis C infection or EP679657, which discloses compounds that serve as ribonucleoside analogues or US2002058635, which discloses purine L-nucleoside compounds, in which both the purine rings and the carbohydrate ring (pentose ring) are either modified, functionalized, or both. So the carbohydrate ring for example must show at least one esterified hydroxy group.

WO 2005/051944 discloses oxetane-containing nucleosides, for the treatment of nucleoside analogue related disorders such as disorders involving cellular proliferation and infection. WO 2006/084281 discloses inhibitors of the E1 activation enzyme that have a sulfonamide moiety.

WO 1998/40384 discloses pyrazolopyrimidinones which are PDE1, 2 and 5 inhibitors and can be employed for the treatment of cardiovascular and cerebrovascular disorders and disorders of the urogenital system.

CH396 924, CH396 925, CH396 926, CH396 927, DE1147234, DE1149013, describe pyrazolopyrimidines which have a coronary-dilating effect and which can be employed for the treatment of disturbances of myocardial blood flow.

US3732225 describes pyrazolopyrimidines which have an anti-inflammatory and blood glucose-lowering effect.

DE2408906 describes styrylpyrazolopyrimidinones which can be employed as antimicrobial and anti-inflammatory agents for the treatment of, for example, oedema.

### OBJECTIVE OF THE INVENTION

Changes in the substitution pattern of pyrazolopyrimidinones result in interesting changes concerning biological activity, respectively changes in the affinity towards different target enzymes.

Therefore it is an objective of the present invention to provide compounds as herein described, in particular in the claims, that effectively modulate PDE9A for the purpose of the development of a medicament, in particular in view of diseases or conditions, the treatment of which is accessible via PDE9A modulation.

It is another objective of the present invention to provide compounds that are useful for the manufacture of a medicament for the treatment of CNS disorders.

Yet another objective of the present invention is to provide compounds which show a favourable safety profile.

Another objective of the present invention is to provide compounds that have a favourable selectively profile in favour of PDE9A inhibition over other PDE family members and other pharmacological targets and by this may provide an advantage.

Yet another objective is to provide a medicament that may not only serve for treatment but might also be used for the prevention or modification of the corresponding disease or condition.

The present invention further provides a pharmaceutical composition comprising a compound as herein described, in particular in the claims and a pharmaceutically acceptable carrier.

The present disclosure further provides a method for the treatment of any of the conditions as described herein in a mammal in need of such treatment, preferably a human, comprising administering to the mammal a therapeutically effective amount of a compound as herein described, in particular in the claims.

The present invention further provides a compound as herein described, in particular in the claims, for use in a method of treatment of the human or animal body by therapy.

### DETAILED DESCRIPTION OF THE PRESENT INVENTION

### Embodiment 1 of the present invention:

A compound of the present invention which is characterised by general formula (I): 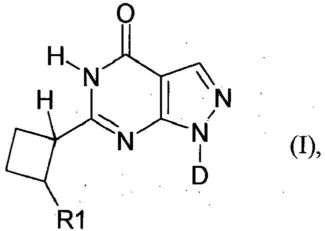 wherein the compound is selected from the group of

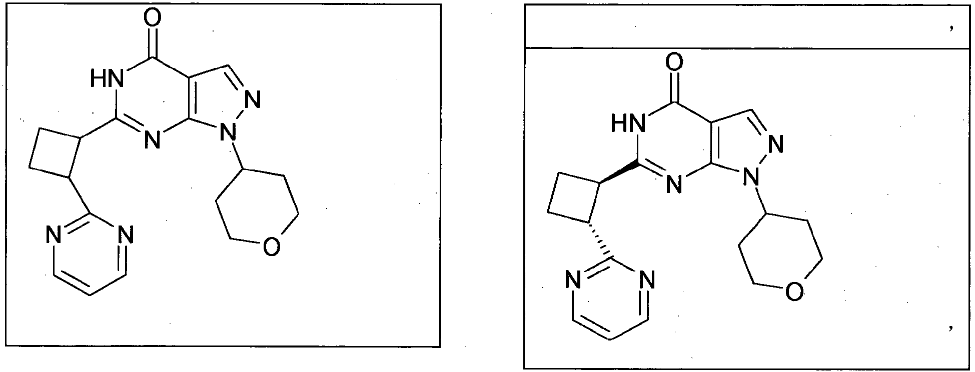

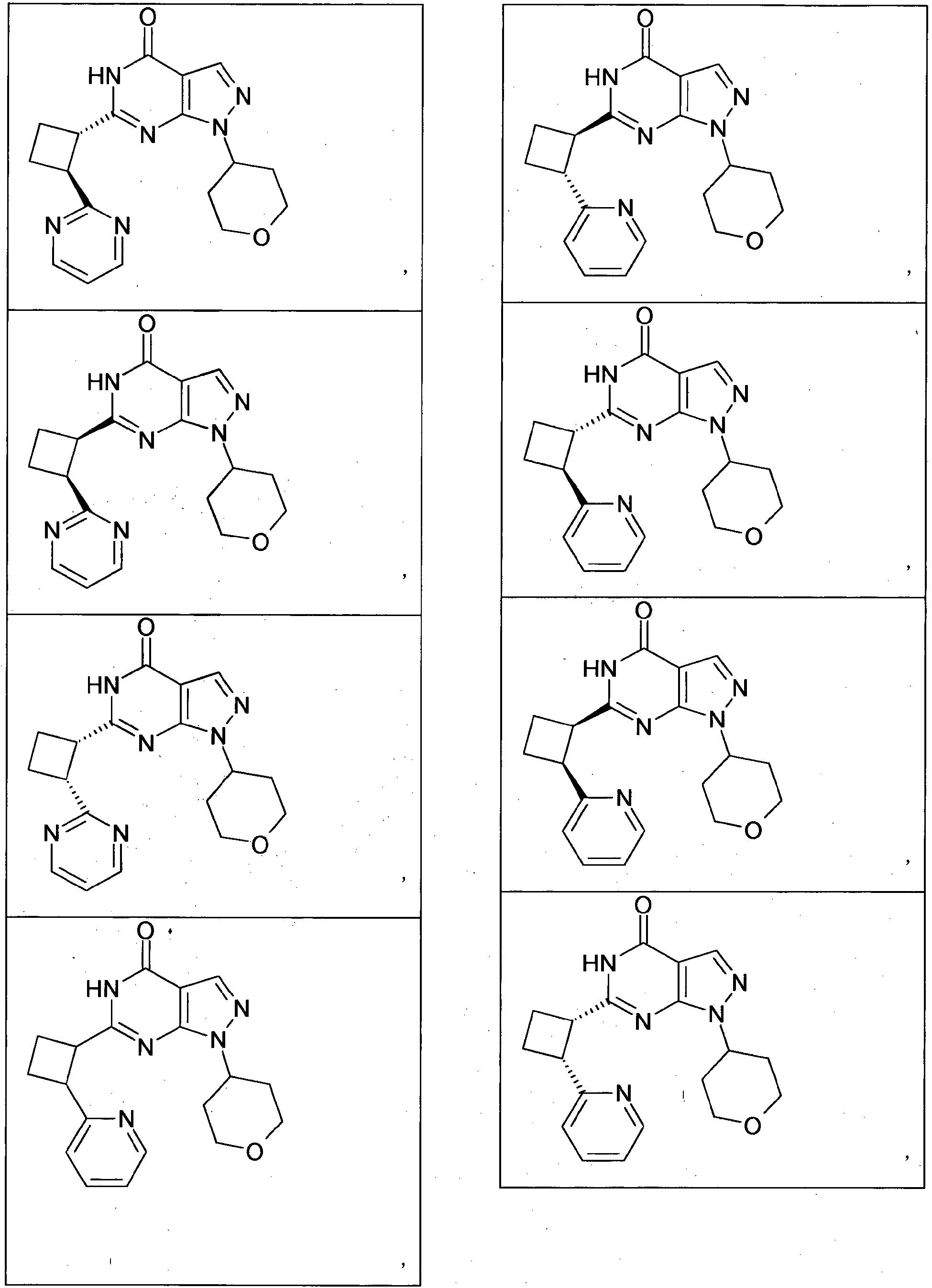

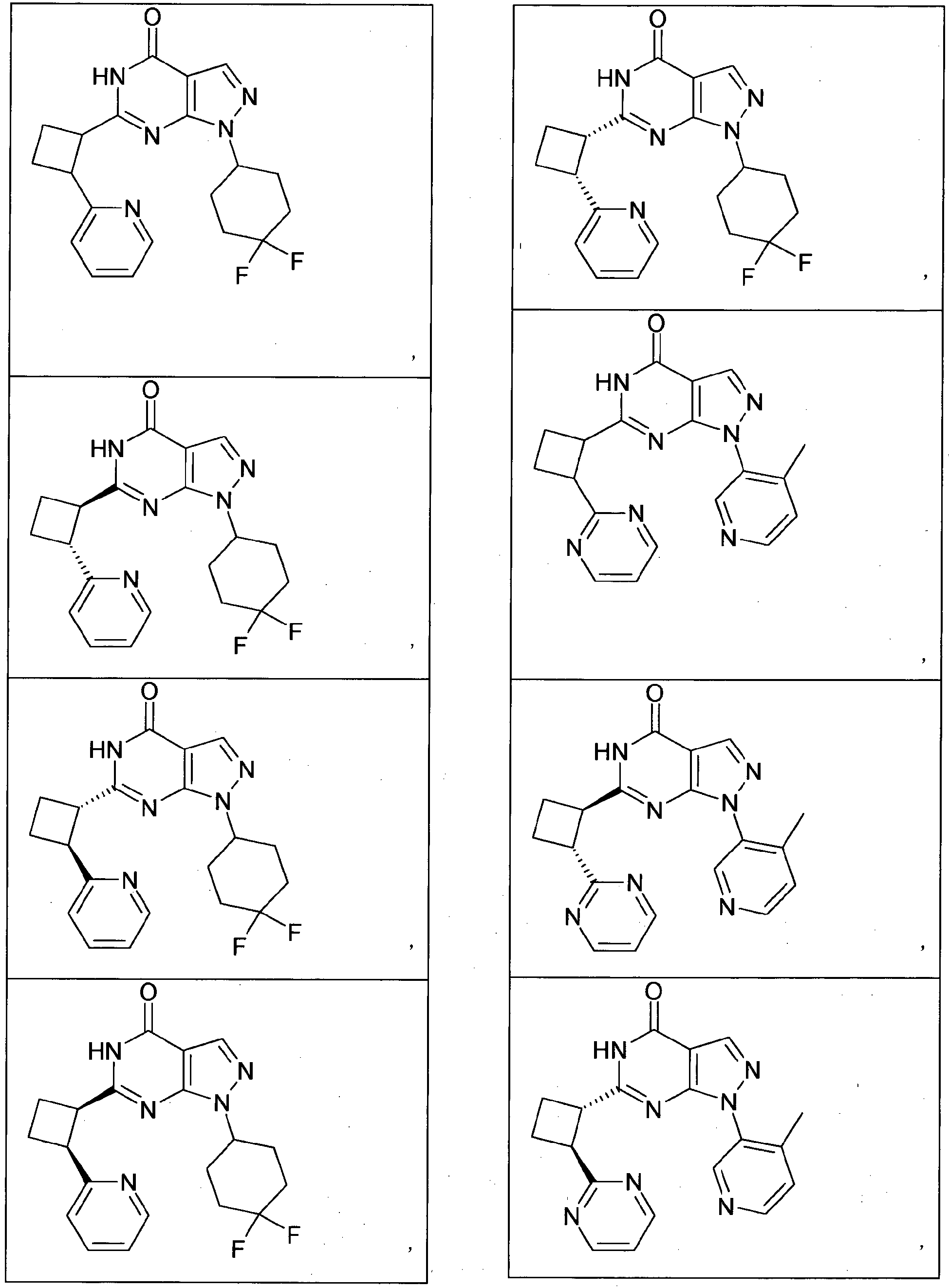

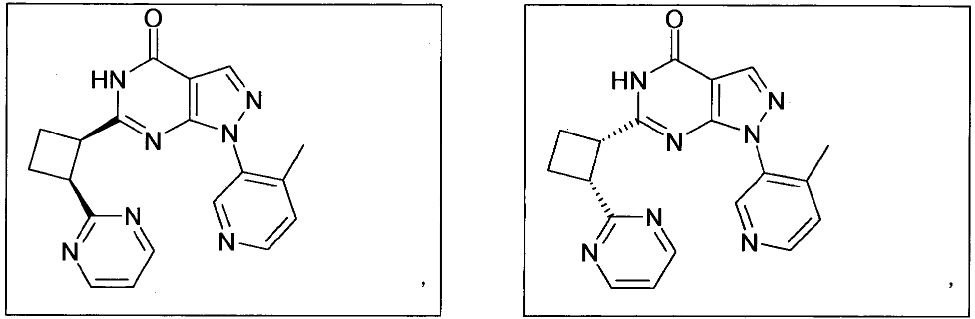

and salts, preferably pharmaceutically acceptable salts thereof.

Through the above group of compounds R¹ and D are defined.

For all exemplified compounds: the configuration of the cycloalkyl group at position 6 of the pyrazolopyrimidinones group with respect to said pyrazolopyrimidinones group and the substituent R¹ may be *cis* or *trans.*

In this respect the compounds of the invention may have the following configurations:

| | |
|---|---|
| *trans* configuration 1 | *trans* configuration 2 |
| 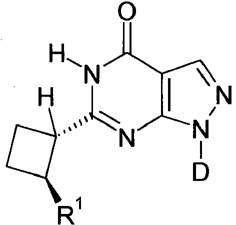 | 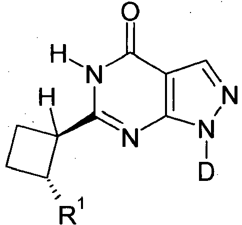 |
| | |
| *cis* configuration 1 | *cis* configuration 2 |
| 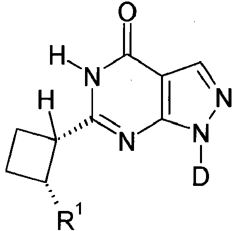 | 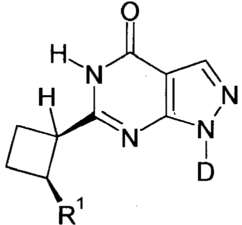 |

whereby R¹ and D are defined as above.

These stereochemically defined embodiments are a further aspect of the invention.

The following table 1 gives an overview about the above listed compounds of the invention, whereby compounds with the same defintion of R¹, R², m, n and D are allocated into compound family groups, which is the group of compounds that have the same general chemical structural formula if no stereochemical properties are considered. Members of these compound families are exemplified in the section **Exemplary embodiments**.

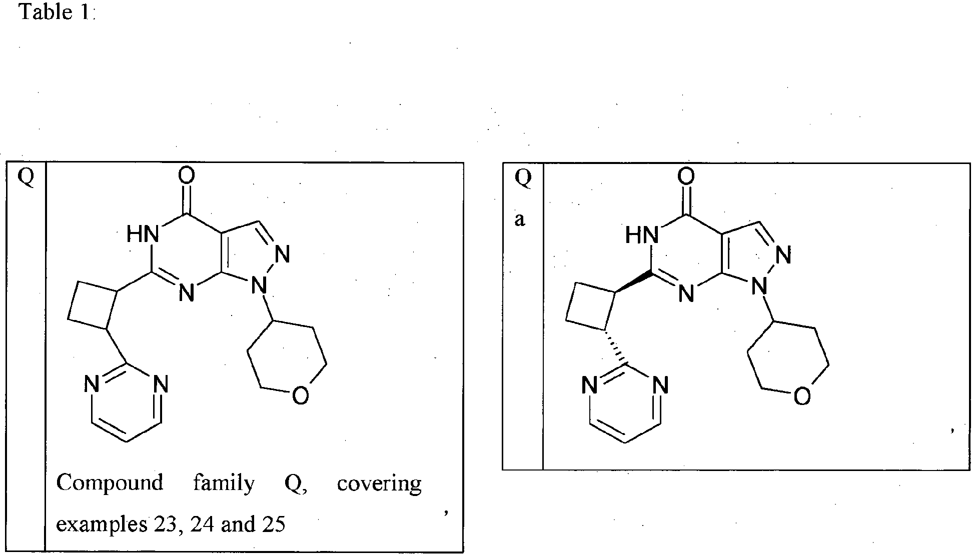

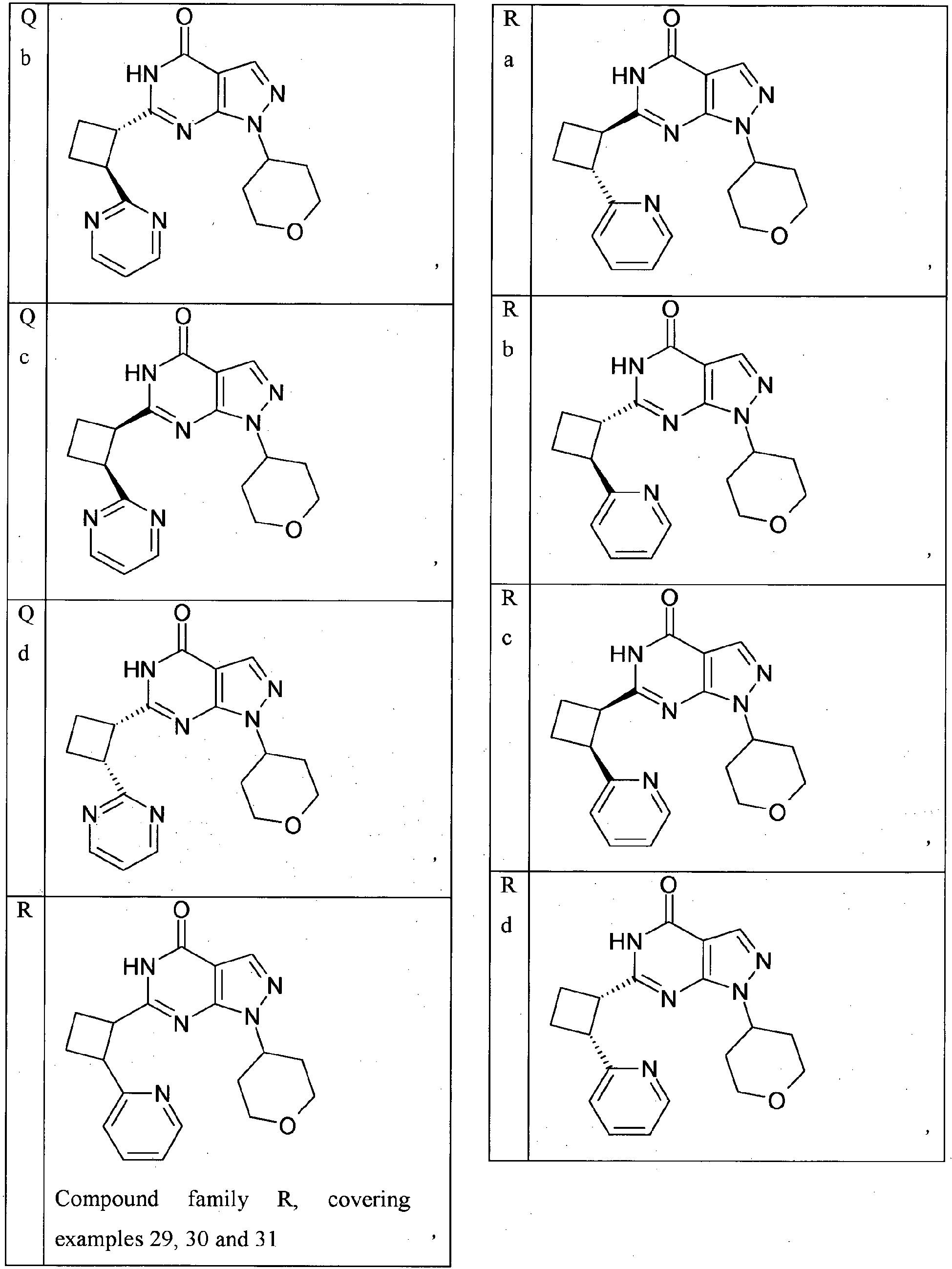

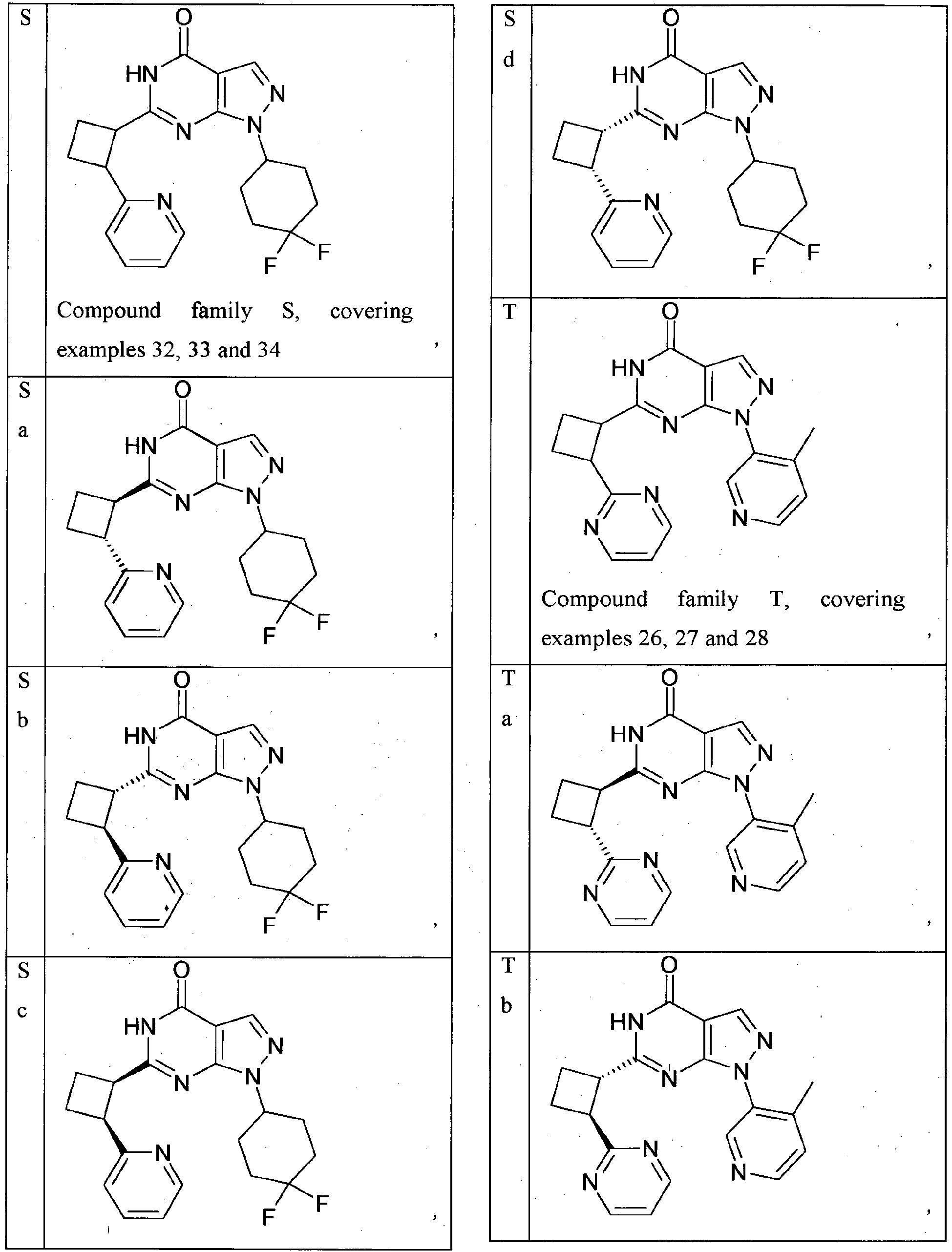

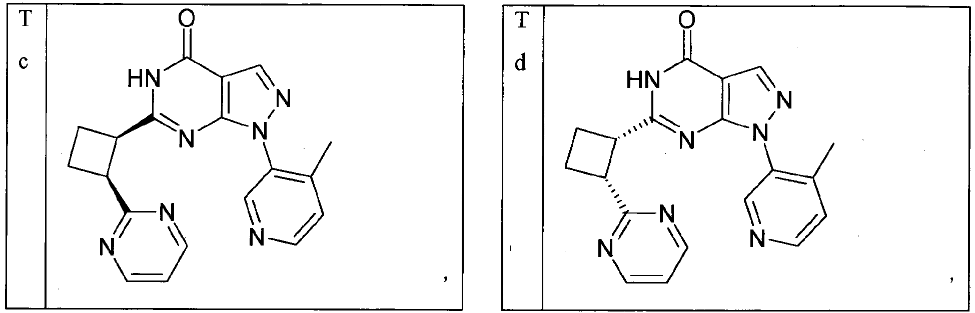

and salts, preferably pharmaceutically acceptable salts thereof, solvates thereof and the solvates of the aforementioned salts thereof.

Within the said group of compounds, compounds that show *trans* configuration with respect to the substitution at the cyclobutyl-group may be preferred over compounds with *cis* configuration. Of the possible *trans* configured compounds one thereof may show advantages in efficacy. The more efficacious a compound the more it is among the preferred compounds. Another criterion which may differentiate preferred compounds according to the invention is the balance of efficacy and safety, such as for example selectivity vs. other PDE family members such as PDE1C.

For one pair of *trans* configured compounds according to the experimental part a single crystal X-ray structure analysis revealed that the absolute stereochemistry of the compound which showed lower efficacy than its enantiomer is R,R. As a consequence thereof absolute stereochemistry of the compound with the higher efficacy is S,S.

For said compound the S,S-configuration is represented by the following structure according to general formula (IId):

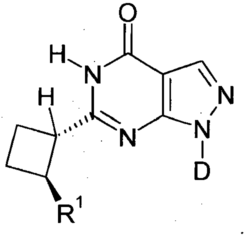

In analogy, one may assume that among the compounds of the invention, such compounds that show the same absolute stereochemistry might be the more active ones compared with the other members of the same compound family. According to the present invention, within the same compound family the more active compounds are preferred over the less active compounds. The compound family is the group of compounds that differ in their chemical structure only with regard to stereochemical properties.

The different stereoisomers are subject to individual embodiments according to the invention:

### Further embodiments of the invention:

**Embodiment 2 of the present invention** concerns the compounds according to embodiment 1 of the present invention which show the following stereochemical properties according to formula (IIa)

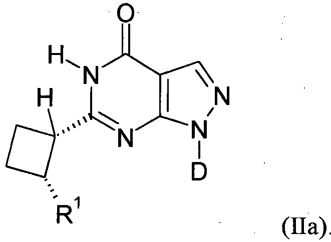

**Embodiment 3 of the present invention** concerns a compound according to embodiment 1 of the present invention, whereby the compound shows the following stereochemical properties according to formula (IIb)

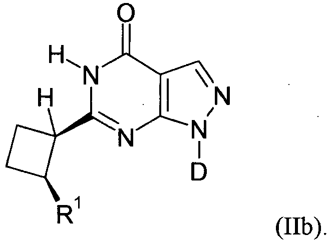

**Embodiment 4 of the present invention** concerns a compound according to embodiment 1 of the present invention, whereby the compound shows the following stereochemical properties according to formula (IIc)

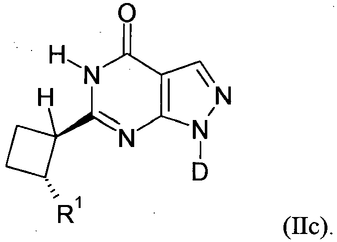

**Embodiment 5 of the present invention** concerns a compound according to embodiment 1 of the present invention, whereby the compound shows the following stereochemical properties according to formula (IId)

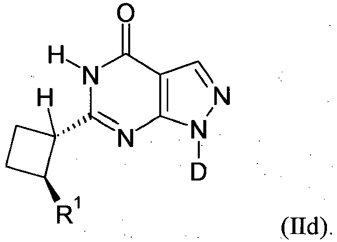

### TERMS AND DEFINITIONS

Terms not specifically defined herein should be given the meanings that would be given to them by a person skilled in the art in light of the disclosure and the context. Examples include that specific substituents or atoms are presented with their 1 or 2 letter code, like H for hydrogen, N for nitrogen, C for carbon, O for oxygen, S for sulphur and the like.

As used in the specification, unless specified to the contrary, the following terms have the meaning indicated and the following conventions are adhered to.

Unless otherwise specified below, conventional definitions of terms control and conventional stable atom valences are presumed and achieved in all formulas and groups.

In general, if terms are specifically defined with a given context, such specific definitions shall prevail over the more general definitions as outlined in this paragraph.

In general, all **"tautomeric forms and isomeric forms and mixtures",** whether individual geometric isomers or optical isomers or racemic or non-racemic mixtures of isomers, of a chemical structure or compound are intended, unless the specific stereochemistry or isomeric form is specifically indicated in the compound name or structure. Specific definitions prevail.

The phrase **"pharmaceutically acceptable"** is employed herein to refer to those compounds, materials, compositions and/or dosage forms which are, within the scope of sound medical judgment, suitable for use in contact with the tissues of human beings or as the case may be of animals without excessive toxicity, irritation, allergic response, or other problem or complication, commensurate with a reasonable benefit/risk ratio.

**"pharmaceutically acceptable salt(s)"** of the compounds according to the invention are subject of the present invention as well. The term **"pharmaceutically acceptable salt(s)"** refers to derivatives of the disclosed compounds wherein the parent compound is modified by making acid or base salts thereof, preferably addition salts. Examples of pharmaceutically acceptable salts include, but are not limited to, mineral or organic acid salts of basic residues/parts of the compounds of the present invention such as aminofunctions; acidic residues/parts within compounds of the present invention may form salts with alkali or organic bases. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric acid, hydrobromic acid, sulfuric acid, sulfamic acid, phosphoric acid, nitric acid and the like; and the salts prepared from organic acids such as acetic acid, propionic acid, succinic acid, glycolic acid, stearic acid, lactic acid, malic acid, tartaric acid, citric acid, ascorbic acid, pamoic acid, maleic acid, hydroxymaleic acid, phenylacetic acid, glutamic acid, benzoic acid, salicylic acid, sulfanilic acid, 2-acetoxybenzoic acid, fumaric acid, toluenesulfonic acid, methanesulfonic acid, ethane disulfonic acid, oxalic acid, isethionic acid and the like.

Physiologically acceptable salts with bases also may include salts with conventional bases such as, by way of example and preferably, alkali metal salts (e.g. sodium and potassium salts), alkaline earth metal salts (e.g. calcium and magnesium salts) and ammonia, organic amines having 1 to 16 C atoms, such as, by way of example and preferably, ethylamine, diethylamine, triethylamine, ethyldiisopropylamine, monoethanolamine, diethanolamine, triethanolamine, dicyclohexylamine, dimethylaminoethanol, procaine, dibenzylamine, N-methyl-morpholine, dehydroabietylamine, arginine, lysine, ethylenediamine and methylpiperidine and the like.

The pharmaceutically acceptable salts of the present invention can be synthesized from the parent compound with basic or acidic properties by conventional chemical methods. Generally, such salts can be prepared by reacting the free acid or base form of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred.

A **"Prodrug"** is considered a compound that is designed to release a biologically active compound according to the present invention *in-vivo* when such prodrug is administered to a mammalian subject. Prodrugs of compounds according to the present invention are prepared by modifying functional groups present in the compound of the invention in such a way that these modifications are retransformed to the original functional groups under physiological conditions. It will be appreciated that prodrugs of the compounds according to the present inventions are subject to the present disclosure as well.

**"Metabolites"** are considered derivatives of the compounds according to the present invention that are formed *in-vivo.* Active metabolites are such metabolites that cause a pharmacological effect. It will be appreciated that metabolites of the compounds according to the present inventions are subject to the present disclosure as well, in particular active metabolites.

Some of the compounds may form **"solvates".** For the purposes of the invention the term "solvates" refers to those forms of the compounds which form, in the solid or liquid state, a complex by coordination with solvent molecules. Hydrates are a specific form of solvates in which the coordination takes place with water. According to the present invention, the term preferably is used for solid solvates, such as amorphous or more preferably crystalline solvates.

**"Scaffold":** The scaffold of the compounds according to the present invention is represented by the following core structure. The numeration of the positions of the ring member atoms is indicated in bold:

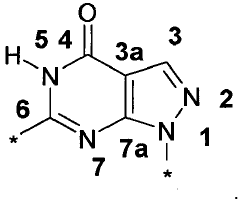

It will be evident for the skilled person in the art, that this scaffold can be described by its tautomeric "enol" form

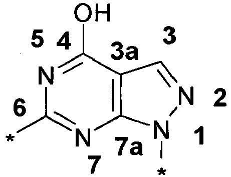

In the context of the present invention both structural representations of the scaffold shall be considered the subject of the present invention, even if only one of the two representatives is presented. Without meant to be limiting or bound, it is believed that for the majority of compounds under ambient conditions and therewith under conditions which are the relevant conditions for a pharmaceutical composition comprising said compounds, the equilibrium of the tautomeric forms lies on the side of the pyrazolopyrimdin-4-one representation. Therefore, all embodiments are presented as pyrazolopyrimdin-4-one-derivatives or more precisely as pyrazolo[3,4-d]pyrimidin-4-one derivatives.

Expressions like **"prevention", "prophylaxis", "prophylactic treatment"** or **"preventive treatment"** used herein should be understood synonymous and in the sense that the risk to develop a condition mentioned hereinbefore is reduced, especially in a patient having elevated risk for said conditions or a corresponding anamnesis. Thus the expression "prevention of a disease" as used herein means the management and care of an individual at risk of developing the disease prior to the clinical onset of the disease. The purpose of prevention is to combat the development of the disease, condition or disorder and includes the administration of the active compounds to prevent or delay the onset of the symptoms or complications and to prevent or delay the development of related diseases, conditions or disorders. Success of said preventive treatment is reflected statistically by reduced incidence of said condition within a patient population at risk for this condition in comparison to an equivalent patient population without preventive treatment.

The expression **"treatment"** or **"therapy"** preferably means therapeutic treatment of (e.g. preferably human) patients having already developed one or more of said conditions in manifest, acute or chronic form, including symptomatic treatment in order to relieve symptoms of the specific indication or causal treatment in order to reverse or partially reverse the condition or to delay the progression of the indication as far as this may be possible, depending on the condition and the severity thereof. Thus the expression "treatment of a disease" as used herein means the management and care of a patient having developed the disease, condition or disorder. The purpose of treatment is to combat the disease, condition, disorder or a symptom thereof. Treatment includes the administration of the active compounds to eliminate or control the disease, condition or disorder as well as to alleviate the symptoms or complications associated with the disease, condition or disorder.

The following schemes shall illustrate generally how to manufacture the compounds of the present invention by way of example. The abbreviated substituents may be as defined for the embodiments of formula (I) if not defined otherwise within the context of the schemes:

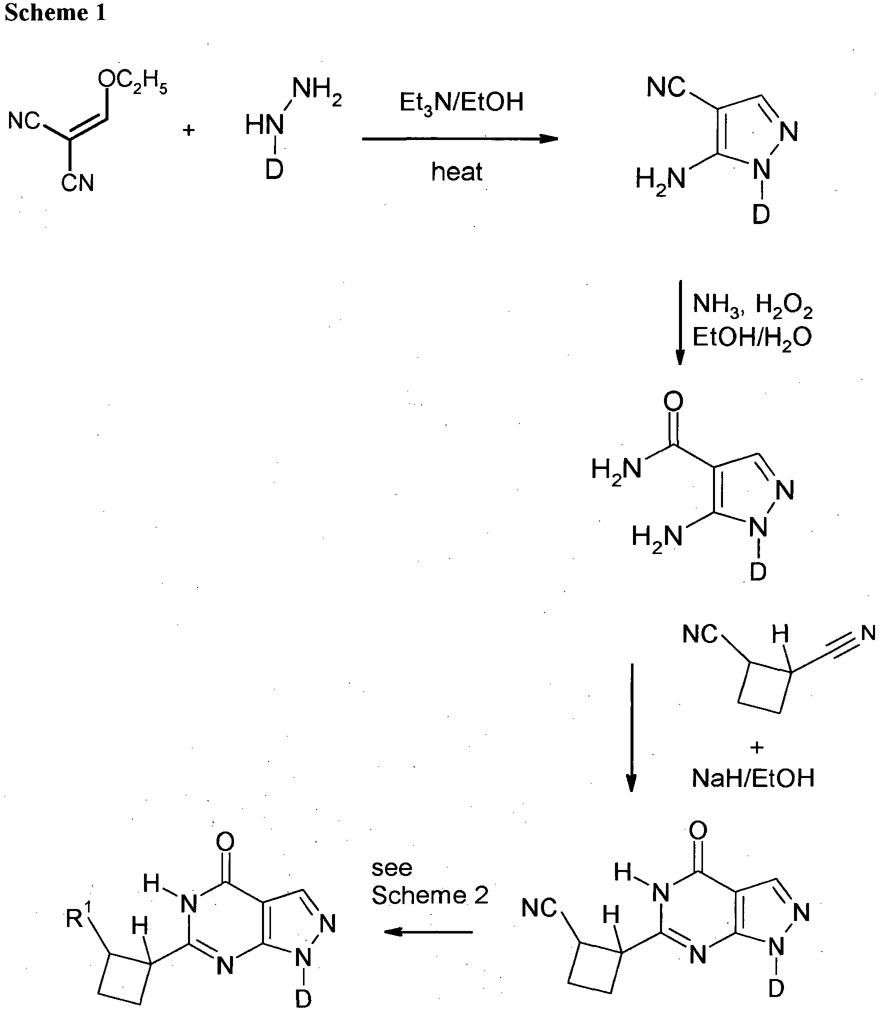

Scheme 1: In a first step 2-ethoxymethylene-malononitrile is condensed with mono-substituted hydrazines by heating in an appropriate solvent like ethanol in the presence of a base (e.g. triethylamine) to form the corresponding 5-amino-1H-pyrazole-4-carbonitriles. These compounds are converted in a second step to the corresponding amides, e.g. by treatment of an ethanolic solution with ammonia (25 % in water) and hydrogen peroxide (35 % in water). 4-oxo-4,5-dihydro-1H-pyrazolo[3,4-d]pyrimidin-6-yl substituted nitriles can be synthesized from dinitriles by heating under basic conditions (e.g. sodium hydride in ethanol) in the third step. The nitrile functional group is further converted to heteroaryl substituents as described in Scheme 2 yielding pyrazolo[3,4-d]pyrimidin-4-ones as final products. [cf., for example, A. Miyashita et al., Heterocycles 1990, 31, 1309ff].

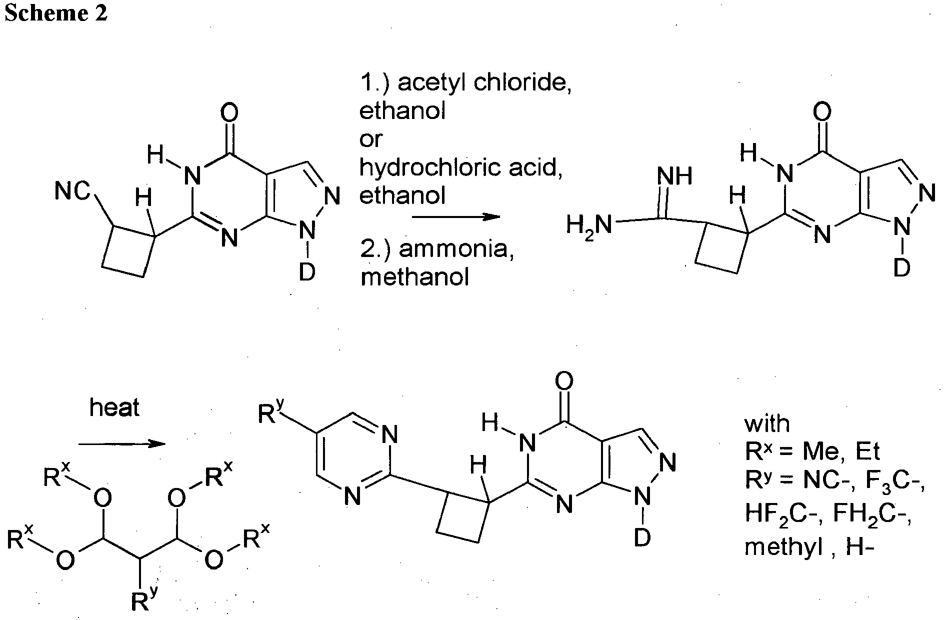

Scheme 2: 4-Oxo-4,5-dihydro-1H-pyrazolo[3,4-d]pyrimidin-6-yl substituted nitriles are mixed with methanol and treated with acetylchloride or, alternatively, mixed with a saturated solution of hydrochloric acid in ethanol. The intermediates are treated in a second step with a solution of ammonia in methanol to form the corresponding amidines. Reaction with a 1,1,3,3-tetraalkoxypropane yields pyrimidin-2-yl substituted pyrazolo[3,4-d]pyrimidin-4-ones as final products.

Further alternative processes for preparing pyrazolo[3,4-d]pyrimidin-4-ones are known in the art and can likewise be employed for synthesizing the compounds of the invention (see, for example: P. Schmidt et al., Helvetica Chimica Acta 1962, 189, 1620ff.).

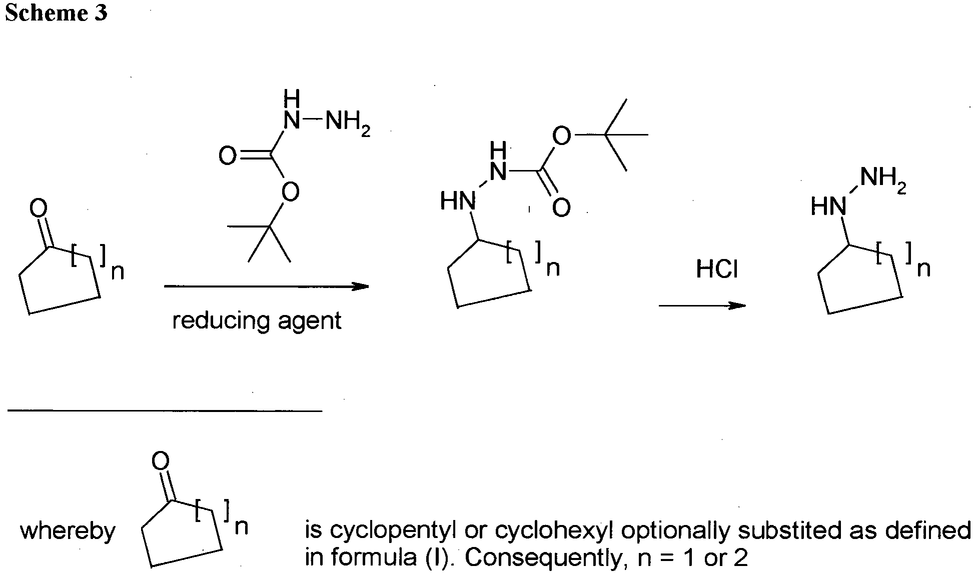

Scheme 3: The mono-substituted hydrazine derivatives, that are used in step 1 of scheme 1 can be prepared by reductive amination of a ketone with hydrazinecarboxylic acid tert-butyl ester followed by a deprotection step as shown in scheme 3 for an D being cyclopentyl or cyclohexyl as defined in general formula (I) [cf., for example, J.W. Timberlake et al., "Chemistry of Hydrazo-, Azo- and Azoxy Groups "; Patai, S., Ed.; 1975, Chapter 4*;* S. C. Hung et al., Journal of Organic Chemistry 1981, 46, 5413-5414*].*

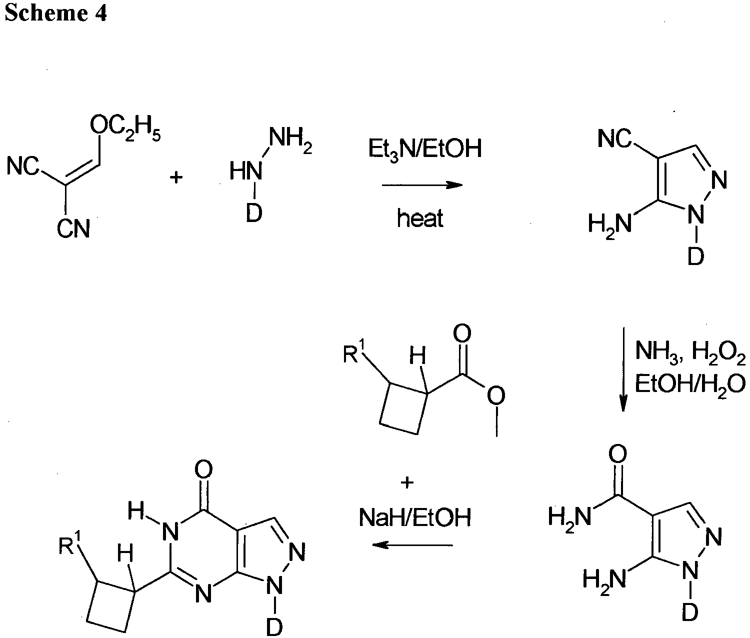

Scheme 4: As described in scheme 1, in a first step 2-ethoxymethylene-malononitrile is condensed with mono-substituted hydrazines by heating in an appropriate solvent like ethanol in the presence of a base (e.g. triethylamine) to form the corresponding 5-amino-1H-pyrazole-4-carbonitriles. These compounds are converted in a second step to the corresponding amides, e.g. by treatment of an ethanolic solution with ammonia (25 % in water) and hydrogen peroxide (35 % in water). In a third step, heating with R¹ and R² substituted cyclobutyl or cyclopentyl carboxylic acid ester under basic conditions (e.g. sodium hydride in ethanol) leads to the final pyrazolo[3,4-d]pyrimidin-4-ones as final products. [cf., for example, A. Miyashita et al., Heterocycles 1990, 31, 1309ff]. This procedure is described in more detail for R¹ being pyridyl in the experimental section (examples 29 to 34).

Further information also can be found in:
- WO 2004/099210 (in particular page 9, last paragraph to page 14, line 8, incorporated by reference),
- with respect to the general manufacture of compounds with D being tetrahydropyranyl more information can be found in WO2009/121919, particularly on page 120 to 125 and the experimental part thereof (herewith incorporated by reference),
- with respect to D being 4,4-difluorocyclohexyl more information can be found in WO 2010/026214, particularly on page 59 to 63 and the experimental part thereof (herewith incorporated by reference),
- and in the experimental part (exemplary embodiments) of this description. The letter in particular with respect to the manufacture of the two building blocks:

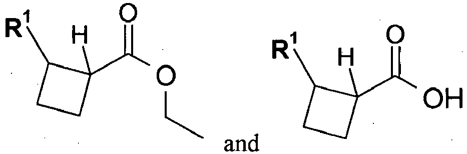

### METHOD OF TREAMENT

The present invention refers to compounds, which are considered effective in the treatment of diseases. The compounds according to the invention are effective and selective inhibitors of phosphodiesterase 9A and can be used for the development of medicaments. Such medicaments shall preferably be used for the treatment of diseases in which the inhibition of PDE9A can provide a therapeutic, prophylactic or disease modifying effect. Preferably the medicaments shall be used to improve perception, concentration, cognition, learning or memory, like those occurring in particular in situations/diseases/syndromes such as: mild cognitive impairment, age-associated learning and memory impairments, age-associated memory losses, vascular dementia, craniocerebral trauma, stroke, dementia occurring after strokes (post stroke dementia), post-traumatic dementia, general concentration impairments, concentration impairments in children with learning and memory problems, Alzheimer's disease, Lewy body dementia, dementia with degeneration of the frontal lobes, including Pick's syndrome, Parkinson's disease, progressive nuclear palsy, dementia with corticobasal degeneration, amyotropic lateral sclerosis (ALS), Huntington's disease, multiple sclerosis, thalamic degeneration, Creutzfeld-Jacob dementia, HIV dementia, epilepsy, temporal lobe epilepsy, schizophrenia, schizophrenia (with dementia), Korsakoff's psychosis or cognitive impairment associated with depression or bipolar disorder.

Another aspect of the present disclosure may concern the treatment of a disease which is accessible by PDE9A modulation, in particular sleep disorders like insomnia or narcolepsy, bipolar disorder, metabolic syndrome, obesity, diabetes mellitus, including type 1 or type 2 diabetes, hyperglycemia, dyslipidemia, impaired glucose tolerance, or a disease of the testes, brain, small intestine, skeletal muscle, heart, lung, thymus or spleen.

Thus the medical aspect of the present invention can be summarised in that it is considered that a compound according to formula (I) or (II) as herein defined, in particular the specifically defined species compounds is used as a medicament.

Such a medicament preferably is for the use in a method for the treatment of a CNS disease.

In an alternative use, the medicament is for the use in a therapeutic or prophylactic method, preferably a therapeutic method, for the treatment of a CNS disease, the treatment of which is accessible by the inhibition of PDE9.

In an alternative use, the medicament is for the use in a therapeutic or prophylactic method, preferably a therapeutic method, for the treatment of a disease that is accessible by the inhibition of PDE9, specifically PDE9A.

In the most preferred alternative use, the medicament is for the use in a therapeutic or prophylactic method, preferably a therapeutic method, for the treatment, amelioration and / or prevention of cognitive impairment being related to perception, concentration, cognition, learning or memory, preferably if such cognitive impairment is associated with a disease or condition as described in this section.

In an alternative use, the medicament is for the use in a therapeutic or prophylactic method, preferably a therapeutic method, for the treatment or the amelioration or prevention of cognitive impairment being related to age-associated learning and memory impairments, age-associated memory losses, vascular dementia, craniocerebral trauma, stroke, dementia occurring after strokes (post stroke dementia), post-traumatic dementia, general concentration impairments, concentration impairments in children with learning and memory problems, Alzheimer's disease, Lewy body dementia, dementia with degeneration of the frontal lobes, including Pick's syndrome, Parkinson's disease, progressive nuclear palsy, dementia with corticobasal degeneration, amyotropic lateral sclerosis (ALS), Huntington's disease, multiple sclerosis, thalamic degeneration, Creutzfeld-Jacob dementia, HIV dementia, epilepsy, temporal lobe epilepsy, schizophrenia, schizophrenia (with dementia), Korsakoff's psychosis or cognitive impairment associated with depression or bipolar disorder.

In an alternative use, the medicament is for the use in a therapeutic or prophylactic method, preferably a therapeutic method, for the treatment of Alzheimer's disease, schizophrenia or cognitive impairment associated with Alzheimer's disease or associated with schizophrenia.

In an alternative use, the medicament is for the use in a therapeutic or prophylactic method, preferably a therapeutic method, for the treatment of sleep disorders, bipolar disorder, metabolic syndrome, obesity, diabetes mellitus, hyperglycemia, dyslipidemia, impaired glucose tolerance, or a disease of the testes, brain, small intestine, skeletal muscle, heart, lung, thymus or spleen.

In a further aspect, the present disclosure relates to the method of treatment or prevention of a condition or disease selected from the above listed groups of conditions and diseases, whereby the method comprises the administration of a therapeutically effective amount of a compound according to the invention in a human being in need thereof.

Another aspect of the invention concerns the compounds of the inventions for use as a medicament in a therapeutic or prophylactic method, preferably a therapeutic method. If indicated the therapeutic method or the medicament is preferably for the treatment of a condition or a disease selected from the group of conditions or a diseases as outlined above in this section which is entitled **"METHOD OF TREATMENT"**.

### PHARMACEUTICAL COMPOSITIONS

Medicaments for administration, which are also subject to the present invention, comprise
- a compound according to the present invention as a or the pharmaceutically active ingredient in a therapeutically effective amount and
- a pharmaceutical carrier.

By "therapeutically effective amount" it is meant that if the medicament is applied via the appropriate regimen adapted to the patient's condition, the amount of said compound of formula (I) will be sufficient to effectively treat, to prevent or to decelerate the progression of the corresponding disease, or otherwise to ameliorate the state of a patient suffering from such a disease. It may be the case that the "therapeutically effective amount" in a mono-therapy will differ from the "therapeutically effective amount" in a combination therapy with another medicament.

The dose range of the compounds of general formula (I) applicable per day may be usually from 0.1 to 5000 mg, preferably from 0.1 to 1000 mg, preferably from 2 to 500 mg, more preferably from 5 to 250 mg, most preferably from 10 to 100 mg. A dosage unit (e.g. a tablet) preferably may contain between 2 and 250 mg, particularly preferably between 10 and 100 mg of the compounds according to the invention.

The actual pharmaceutically effective amount or therapeutic dosage will depend on factors known by those skilled in the art such as age, weight, gender or other condition of the patient, route of administration, severity of disease and the like.

The compounds according to the invention may be administered by oral, parenteral (intravenous, intramuscular etc.), intranasal, sublingual, inhalative, intrathecal, topical or rectal route. Suitable preparations for administering the compounds according to the present invention include for example patches, tablets, capsules, pills, pellets, dragees, powders, troches, suppositories, liquid preparations such as solutions, suspensions, emulsions, drops, syrups, elixirs, or gaseous preparations such as aerosols, sprays and the like. The content of the pharmaceutically active compound(s) should be in the range from 0.05 to 90 wt.-%, preferably 0.1 to 50 wt.-% of the composition as a whole. Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number of layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavour enhancer, e.g. a flavouring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions may be prepared in the usual way, e.g. with the addition of isotonic agents, preservatives such as p-hydroxybenzoates or stabilisers such as alkali metal salts of ethylenediamine-tetra-acetic acid, optionally using emulsifiers and/or dispersants, while if water shall be used as diluent, for example, organic solvents may optionally be used as solubilisers or dissolving aids and the solutions may be transferred into injection vials or ampoules or infusion bottles.

Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof.

Excipients which may be used include, for example, water, pharmaceutically acceptable organic solvents such as paraffins (e.g. petroleum fractions), vegetable oils (e.g. groundnut or sesame oil), mono- or polyfunctional alcohols (e.g. ethanol or glycerol), carriers such as e.g. natural mineral powders (e.g. kaolins, clays, talc, chalk), synthetic mineral powders (e.g. highly dispersed silicic acid and silicates), sugars (e.g. cane sugar, lactose and glucose), emulsifiers (e.g. lignin, spent sulphite liquors, methylcellulose, starch and polyvinylpyrrolidone) and lubricants (e.g. magnesium stearate, talc, stearic acid and sodium lauryl sulphate).

For oral use the tablets may contain, in addition to the carriers specified, additives such as sodium citrate, calcium carbonate and dicalcium phosphate together with various additional substances such as starch, preferably potato starch, gelatine and the like. Lubricants such as magnesium stearate, sodium laurylsulphate and talc may also be used to produce the tablets. In the case of aqueous suspensions the active substances may be combined with various flavour enhancers or colourings in addition to the abovementioned excipients.

The dosage of the compounds according to the invention is naturally highly dependent on the method of administration and the complaint which is being treated.

### COMBINATIONS WITH OTHER ACTIVE SUBSTANCES

In another aspect the present invention relates to a combination therapy in which a compound according to the present invention is administered together with another active compound. Accordingly, the invention also refers to pharmaceutical formulations that provide such a combination of pharmaceutically active ingredients, whereby one of which is a compound of the present invention. Such combinations may be fixed dose combinations (the pharmaceutically active ingredients that are to be combined are subject of the same pharmaceutical formulation) or free dose combinations (the pharmaceutically active ingredients are in separate pharmaceutical formulations).

Consequently, a further aspect of the present invention refers to a combination of each of the compounds of the present invention, preferably at least one compound according to the present invention, with another active compound for example selected from the group of beta-secretase inhibitors; gamma-secretase inhibitors; gamma-secretase modulators; amyloid aggregation inhibitors such as e.g. alzhemed; directly or indirectly acting neuroprotective and/or disease-modifying substances; anti-oxidants, such as e.g. vitamin E , ginko biloba or ginkolide; anti-inflammatory substances, such as e.g. Cox inhibitors, NSAIDs additionally or exclusively having Aβ (Abeta) lowering properties; HMG-CoA reductase inhibitors, such as statins; acetylcholine esterase inhibitors, such as donepezil, rivastigmine, tacrine, galantamine; NMDA receptor antagonists such as e.g. memantine; AMPA receptor agonists; AMPA receptor positive modulators, AMPkines, glycine transporter 1 inhibitors; monoamine receptor reuptake inhibitors; substances modulating the concentration or release of neurotransmitters; substances inducing the secretion of growth hormone such as ibutamoren mesylate and capromorelin; CB-1 receptor antagonists or inverse agonists; antibiotics such as minocyclin or rifampicin; PDE1, PDE2, PDE4, PDE5 and / or PDE10 inhibitors, GABAA receptor inverse agonists; GABAA alpha5 receptor inverse agonists; GABAA receptor antagonists; nicotinic receptor agonists or partial agonists or positive modulators; alpha4beta2 nicotinic receptor agonists or partial agonists or positive modulators; alpha7 nicotinic receptor agonists or partial agonists; histamine receptor H3 antagonists; 5-HT4 receptor agonists or partial agonists; 5-HT6 receptor antagonists; alpha2-adrenoreceptor antagonists, calcium antagonists; muscarinic receptor M1 agonists or partial agonists or positive modulators; muscarinic receptor M2 antagonists; muscarinic receptor M4 antagonists; metabotropic glutamate receptor 5 positive allosteric modulators; metabotropic glutamate receptor 2 antagonists; metabotropic glutamate receptor 2/3 agonists; metabotropic glutamate receptor 2 positive allosteric modulators and other substances that modulate receptors or enzymes in a manner such that the efficacy and/or safety of the compounds according to the invention is increased and/or unwanted side effects are reduced.

This invention further relates to pharmaceutical compositions containing one or more, preferably one active substance. At least one active substance is selected from the compounds according to the invention and/or the corresponding salts thereof. Preferably the composition comprises only one such active compound. In case of more than one active compound the other one can be selected from the aforementioned group of combination partners such as alzhemed, vitamin E, ginkolide, donepezil, rivastigmine, tacrine, galantamine, memantine, ibutamoren mesylate, capromorelin, minocyclin and/or rifampicin. Optionally the composition comprises further ingredients such as inert carriers and/or diluents.

The compounds according to the invention may also be used in combination with immunotherapies such as e.g. active immunisation with Abeta or parts thereof or passive immunisation with humanised anti-Abeta antibodies or antibody fragments for the treatment of the above mentioned diseases and conditions.

The compounds according to the invention also may be combined with Dimebon.

The compounds according to the invention also may be combined with antidepressants like amitriptyline imipramine hydrochloride (TOFRANIL), imipramine maleate (SURMONTIL), lofepramine, desipramine (NORPRAMIN), doxepin (SINEQUAN, ZONALON), trimipramine (SURMONTIL).

Or the compounds according to the invention also may be combined with serotonin (5-HT) reuptake inhibitors such as alaproclate, citalopram (CELEXA, CIPRAMIL) escitalopram (LEXAPRO, CIPRALEX), clomipramine (ANAFRANIL), duloxetine (CYMBALTA), femoxetine (MALEXIL), fenfluramine (PONDIMIN), norfenfluramine, fluoxetine (PROZAC), fluvoxamine (LUVOX), indalpine, milnacipran (IXEL), paroxetine (PAXIL, SEROXAT), sertraline (ZOLOFT, LUSTRAL), trazodone (DESYREL, MOLIPAXIN), venlafaxine (EFFEXOR), zimelidine (NORMUD, ZELMID), bicifadine, desvenlafaxine (PRISTIQ), brasofensme and tesofensine.

The combinations according to the present invention may be provided simultaneously in one and the same dosage form, i.e. in form of a combination preparation, for example the two components may be incorporated in one tablet, e. g. in different layers of said tablet. The combination may be also provided separately, in form of a free combination, i.e. the compounds of the present invention are provided in one dosage form and one or more of the above mentioned combination partners is provided in another dosage form. These two dosage forms may be equal dosage forms, for example a co-administration of two tablets, one containing a therapeutically effective amount of the compound of the present invention and one containing a therapeutically effective amount of the above mentioned combination partner. It is also possible to combine different administration forms, if desired. Any type of suitable administration forms may be provided.

The compound according to the invention, or a physiologically acceptable salt thereof, in combination with another active substance may be used simultaneously or at staggered times, but particularly close together in time. If administered simultaneously, the two active substances are given to the patient together; if administered at staggered times the two active substances are given to the patient successively within a period of less than or equal to 12, particularly less than or equal to 6 hours.

The dosage or administration forms are not limited, in the context of the present invention any suitable dosage form may be used. For example, the dosage forms may be selected from solid preparations such as patches, tablets, capsules, pills, pellets, dragees, powders, troches, suppositories, liquid preparations such as solutions, suspensions, emulsions, drops, syrups, elixirs, or gaseous preparations such as aerosols, sprays and the like.

The dosage forms are advantageously formulated in dosage units, each dosage unit being adapted to supply a single dose of each active component being present. Depending from the administration route and dosage form the ingredients are selected accordingly.

The dosage for the above-mentioned combination partners may be expediently 1/5 of the normally recommended lowest dose up to 1/1 of the normally recommended dose.

The dosage forms are administered to the patient for example 1, 2, 3, or 4 times daily depending on the nature of the formulation. In case of retarding or extended release formulations or other pharmaceutical formulations, the same may be applied differently (e.g. once weekly or monthly etc.). It is preferred that the compounds of the invention be administered either three or fewer times, more preferably once or twice daily.

### EXAMPLES

### PHARMACEUTICAL COMPOSITIONS

Examples which might illustrate possible pharmaceutical formulations, without being meant to be limiting:
The term "active substance" denotes one or more compounds according to the invention including the salts thereof. In the case of one of the aforementioned combinations with one or more other active substances the term "active substance" may also include the additional active substances.

### Example A

Tablets containing 100 mg of active substance

### Composition: tablet

| | |
|---|---|
| active substance | 100.0 mg |
| lactose | 80.0 mg |
| corn starch | 34.0 mg |
| polyvinylpyrrolidone | 4.0 mg |
| magnesium stearate | 2.0 mg |
| | 220.0 mg |

### Example B

Tablets containing 150 mg of active substance

### Composition: tablet

| | |
|---|---|
| active substance | 150.0 mg |
| powdered lactose | 89.0 mg |
| corn starch | 40.0 mg |
| colloidal silica | 10.0 mg |
| polyvinylpyrrolidone | 10.0 mg |
| magnesium stearate | 1.0 mg |
| | 300.0 mg |

### Example C

Hard gelatine capsules containing 50 mg of active substance

| | |
|---|---|
| active substance | 150.0 mg |
| lactose | 87.0 mg |
| corn starch (dried) | 80.0 mg |
| magnesium stearate | 3.0 mg |
| | 320.0 mg |

### Example D

### Composition: suppository

| | |
|---|---|
| active substance | 150.0 mg |
| polyethyleneglycol 1500 | 550.0 mg |
| polyethyleneglycol 6000 | 460.0 mg |
| polyoxyethylene sorbitan monostearate | 840.0 mg |
| | 2000.0 mg |

### Example E

### Composition: ampoules containing 10 mg active substance

| | |
|---|---|
| active substance | 10.0 mg |
| 0.01 N hydrochloric acid q.s. double-distilled water ad | 2.0 mL |

### Example F

### Composition: ampoules containing 50 mg of active substance

| | |
|---|---|
| active substance | 50.0 mg |
| 0.01 N hydrochloric acid q.s. double-distilled water ad | 10.0 mL |

The preparation of any the above mentioned formulations can be done following standard procedures.

### BIOLOGICAL ASSAY

The *in-vitro* effect of the compounds of the invention can be shown with the following biological assays.

### PDE9A2 assay protocol:

The PDE9A2 enzymatic activity assay was run as scintillation proximity assay (SPA), in general according to the protocol of the manufacturer (GE Healthcare, former Amersham Biosciences, product number: TRKQ 7100).

As enzyme source, lysate (PBS with 1 % Triton X-100 supplemented with protease inhibitors, cell debris removed by centrifugation at 13.000 rpm for 30 min) of SF 9 cell expressing the human PDE9A2 was used. The total protein amount included in the assay varied upon infection and production efficacy of the SF9 cells and lay in the range of 0.1 - 100 ng.

In general, the assay conditions were as follows:
- total assay volume: 40 microlitre
- protein amount: 0.1 - 50 ng
- substrate concentration (cGMP): 20 nanomolar; ∼1 mCi/l
- incubation time: 60 min at room temperature
- final DMSO concentration: 0.2 - 1 %

The assays were run in 384-well format. The test reagents as well as the enzyme and the substrate were diluted in assay buffer. The assay buffer contained 50 mM Tris, 8.3 mM MgCl₂, 1.7 mM EGTA, 0.1 % BSA, 0.05 % Tween 20; the pH of assay buffer was adjusted to 7.5. The reaction was stopped by applying a PDE9 specific inhibitor (e.g. compounds according to WO 2004/099210 or WO 2004/099211, like one of the enantiomeres of example 37, e.g. 1-(2-Chlorophenyl)-6-[(2R)-3,3,3-trifluoro-2-methyl-propyl]-1,5-dihydro-4H-pyrazolo[3,4-d]pyrimidine-4-one) in excess.

### References:

Wunder F, Tersteegen A, Rebmann A, Erb C, Fahrig T, Hendrix M. Characterization of the first potent and selective PDE9 inhibitor using a cGMP reporter cell line. Molecular Pharmacology. 2005 Dec;68(6):1775-81.

van der Staay FJ, Rutten K, Barfacker L, Devry J, Erb C, Heckroth H, Karthaus D, Tersteegen A, van Kampen M, Blokland A, Prickaerts J, Reymann KG, Schröder UH, Hendrix M. The novel selective PDE9 inhibitor BAY 73-6691 improves learning and memory in rodents. Neuropharmacology. 2008 Oct;55(5):908-18.

### PDE1C assay protocol:

The assay was run in an analogous manner to the PDE9A2 assay, with the following differences: instead of PDE9A2, PDE1C was used and the assay buffer contained in addition 50 nM Calmodulin, 3 mM CaCl₂. The reaction can be stopped by applying the same inhibitor than the one that is outlined above (1-(2-Chlorophenyl)-6-[(2R)-3,3,3-trifluoro-2-methylpropyl]-1,5-dihydro-4H-pyrazolo[3,4-d]pyrimidine-4-one).

### Determination of IC₅₀:

IC₅₀ can be calculated with GraphPadPrism or other suited software setting the positive control as 100 and the negative control as 0. For calculation of IC₅₀ dilutions of the test compounds (substrates) are to be selected and tested following the aforementioned protocol.

### Data

In the following IC₅₀ values for PDE9A2 inhibition [nanomolar (nM)] illustrate that the compounds according to the present invention inhibit PDE9, specifically PDE9A2. This evidences that the compounds provide useful pharmacological properties. The examples are not meant to be limiting.

The table also provides selectivity values (Selectivity) that show a preference of the compounds for PDE9A versus PDE1C. Selectivity is the ratio (IC₅₀ for PDE1C inhibition [nanomolar (nM)]) / (IC₅₀ for PDE9A2 inhibition [nanomolar (nM)]).

The example numbers refer to the final examples as outlined in the section **Exemplary embodiments** and as defined by the above compound family table (table 2).

All data can be measured according to the procedure described herein. The definition enantiomer 1 or enantiomer 2 is related to the elution orders of enantiomers in chiral SFC and chiral HPLC.

**Table 2:**

| Compound family | Example No. | IC₅₀ PDE9A2 [nanomolar] | Selectivity |
|---|---|---|---|
| Q | 23* | 23 | 187 |
| Q | 24 (enantiomer 1) | 218 | 8.9 |
| Q | 25 (enantiomer 2) | 7 | 197 |
| R | 29* | 11 | 117 |
| R | 30 (enantiomer 1) | 304 | 4.95 |
| R | 31 (enantiomer 2) | 7 | 186 |
| S | 32* | 7 | 117 |
| S | 33 (enantiomer 1) | 4 | 181 |
| S | 34 (enantiomer 2) | 388 | 1.68 |
| T | 26* | 32 | >400 |
| T | 27 (enantiomer 1) | 11 | 250 |
| T | 28 (enantiomer 2) | 360 | 7 |

| | | | |
|---|---|---|---|
| * trans racemic mixture | | | |

### In-vivo effect:

It is believed that the positive *in-vitro* efficacy results of the compounds of the present invention translate in positive *in-vivo* efficacy.

The *in-vivo* effect of the compounds of this invention can be tested in the Novel Object Recognition test according to the procedure of Prickaerts et al. (Neuroscience 2002, 113, 351-361), the social recognition test or the T-maze spontaneous alternation test according to the procedures described by van der Staay et al. (Neuropharmacology 2008, 55, 908-918). For further information concerning biological testing one is also referred to these two citations.

Besides the inhibition property toward the target PDE9, compounds according to the present invention may provide further advantageous pharmacokinetic properties.

E.g. compounds according to the invention may show one or more advantages in the area of safety, balanced metabolism, low risk of causing drug - drug interaction and/or balanced clearance.

Compounds also might show one or more additional or alternative advantages in the area of bioavailability, high fraction absorbed, blood brain transport properties, a favourable (e.g. high mean) residence time (mrt), favourable exposure in the effect compartment and so on.

### CHEMICAL MANUFACTURE

In the following compounds and their manufacture are presented some of which are subject to the present invention some of which are for additional illustration. Compounds 23 to 34 are subject of the present invention.

### Abbreviations:

- Burgess-reagent: (methoxycarbonylsulfamoyl)-triethylammonium-N-betain
- Lawesson's reagent: 2,4-bis-(4-methoxy-phenyl)-[1,3,2,4]dithiadiphosphetane 2,4-disulfide
- APCI: Atmospheric pressure chemical ionization
- ACN: acetonitrile
- CDI: 1,1'-carbonyldiimidazole
- DEA: diethylamine
- DIPEA: diisopropylethylamine
- DME: 1,2-dimethoxyethane
- DMF: dimethylformamide
- ESI: electrospray ionization (in MS)
- EtOH: ethanol
- Exp.: example
- h: hour(s)
- HATU: O-(7-azabenzotriazol-1-yl)-N,N,N',N'-tetramethyluronium hexafluorophosphate
- HPLC: high performance liquid chromatography
- HPLC-MS: coupled high performance liquid chromatography-mass spectrometry
- M: molar (mol/L)
- MeOH: methanol
- min: minutes
- MS: mass spectrometry
- NMP: 1-methyl-2-pyrrolidinone
- Rₜ: retention time (in HPLC)
- SFC: supercrticial fluid chromatography
- TBTU: O-(benzotriazol-1-yl)-N,N,N',N'-tetramethyluronium tetrafluoroborate
- TFA: trifluoroacetic acid
- THF: tetrahydrofuran
- TLC: thin-layer chromatography

### LC-MS methods:

### Method 1

MS apparatus type: Waters Micromass ZQ; HPLC apparatus type: Waters Alliance 2695, Waters 2996 diode array detector; column: Varian Microsorb 100 C18, 30 x 4.6 mm, 3.0 µm; eluent A: water + 0.13 % TFA, eluent B: ACN; gradient: 0.0 min 5 % B → 0.18 min 5 % B → 2.0 min 98 % B → 2.2 min 98 % B → 2.3 min 5 % B → 2.5 min 5 % B; flow rate: 3.5 mL/min; UV detection: 210-380 nm.

### Method 2

MS apparatus type: Waters Micromass ZQ; HPLC apparatus type: Waters Alliance 2695, Waters 2996 diode array detector; column: Varian Microsorb 100 C18, 30 x 4.6 mm, 3.0 µm; eluent A: water + 0.13 % TFA, eluent B: MeOH; gradient: 0.0 min 5 % B → 0.35 min 5 % B → 3.95 min 100 % B → 4.45 min 100 % B → 4.55 min 5 % B → 4.9 min 5 % B; flow rate: 2.4 mL/min; UV detection: 210-380 nm.

### Method 3

MS apparatus type: Waters Micromass ZQ; HPLC apparatus type: Waters Alliance 2695, Waters 2996 diode array detector; column: Varian Microsorb C18, 20 x 4.6 mm, 5.0 µm; eluent A: water + 0.15 % TFA, eluent B: MeOH; gradient: 0.0 min 5 % B → 0.25 min 5 % B → 1.90 min 100 % B → 2,05 min 100 % B → 2.15 min 5 % B → 2.25 min 5 % B; flow rate: 5.2 mL/min; UV detection: 210-400 nm.

### Method 1E hydro

Instrument: LC/MS ThermoFinnigan. Hplc Surveyor DAD, MSQ Quadrupole; column: Synergi Hydro-RP80A, 4 um, 4.60 x 100 mm; eluent A: 90% water + 10% acetonitrile + ammonium formate 10 mM; eluent B = ACN 90%+10% H₂O + NH₄COOH 10 mM; gradient: A (100) for 1.5 min, then to B (100) in 10 min for 1.5 min; flow rate: 1.2 mL/min; UV Detection: 254nm; Ion source: APCI.

### Chiral SFC methods:

### Method 4

SFC apparatus type: Berger "Analytix"; column: Daicel IC, 250 mm x 4.6 mm, 5.0 µm; eluent: CO₂ / 25 % MeOH / 0.2 % DEA (isocratic); flow rate: 4.0 mL/min, 10 min; temperature: 40°C; UV detection: 210/220/254 nm.

### Method 5

SFC apparatus type: Berger "Analytix"; column: Daicel ADH, 250 mm x 4.6 mm, 5.0 µm; eluent: CO₂ / 25 % MeOH / 0.2 % DEA (isocratic); flow rate: 4.0 mL/min, 10 min; temperature: 40°C; UV detection: 210/220/254 nm.

### Chiral HPLC methods:

### Method 6:

HPLC apparatus type: Agilent 1100; column: Daicel chiralcel OJ-H, 250 mm x 4.6 mm, 5.0 µm,; eluent: hexane/EtOH80:20; flow rate: 1 mL/min, Temperature: 25°C; UV Detection: variable (200- 500 nm).

### Method 6.1:

HPLC apparatus type: Agilent 1100; column: Daicel chiralcel OJ-H, 250 mm x 4.6 mm, 5.0 µm,; eluent: hexane/EtOH 85:15; flow rate: 1 mL/min, Temperature: 25°C; UV Detection: variable (200- 500 nm).

### Method 7:

HPLC apparatus type: Agilent 1100; column: Chiralpak AD-H, 250 mm x 4.6 mm, 5.0 µm,; eluent: hexane/isopropanol 80:20; flow rate: 1 mL/min, Temperature: 25°C; UV Detection: variable (200- 500 nm).

HPLC apparatus type: Agilent 1100; column: Chiralpak AD-H, 250 mm x 4.6 mm, 5.0 µm,; eluent: hexane/isopropanol 80:20; flow rate: 1 mL/min, Temperature: 25°C; UV Detection: variable (200- 500 nm).

### Microwave heating:

- Discover® CEM instruments, equipped with 10 and 35 mL vessels;
- Biotage Initiator Sixty.

### General comment concerning the presentation of the structures

Compounds with stereogenic centre(s): The structures depicted in the experimental section below will not necessarily show all the stereochemical possibilities of the compounds but only one. However, in such cases a term like "trans - racemic mixture" or "cis-racemic mixture" is added next to the depicted structure in order to indicate the other stereochemical options.

An example is given below. The presented structural formula is

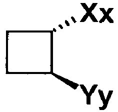

### trans - racemic mixture

The added term "trans-racemic mixture" points to the second stereochemical option:

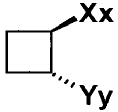

Thus, the manufactured compound is a mixture of

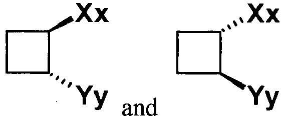

This principle applies to other depicted structures as well.

### Starting compounds:

### Example 1A (trans - racemic mixture)

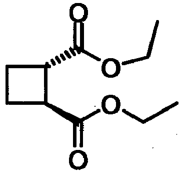

### trans - racemic mixture

2.00 g (13.9 mmol) trans-cyclobutan-1,2-dicarboxylic acid were mixed with 16 mL EtOH at 0°C and 2.21 mL (30.5 mmol) thionylchloride were slowly added. The mixture was allowed to warm to room temperature and stirred for 1h. The solvent was removed under reduced pressure and the product was filtered through a pad of activated basic alumina. 2.71 g (98 %) of the product were obtained.
HPLC-MS (Method 1): Rₜ = 1.34 min
MS (ESI pos): m/z = 201 (M+H)⁺

The following example was synthesized in analogy to the preparation of Example 1A, using the corresponding diacid as starting material.

| Example | structure | starting material | Rₜ [min] | MS (ESI pos, m/z) |
|---|---|---|---|---|
| Exp. 1B cis - racemic mixture | 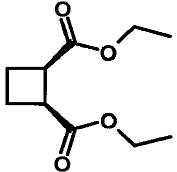 | 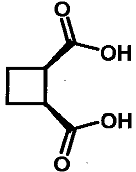 | 1.12 (Method 3) | 201 (M+H)⁺ |

### Example 2A (racemic mixture)

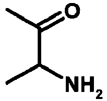

8.00 g (89.7 mmol) 2-amino-propionic acid were mixed with 88.0 mL (0.93 mol) acetic anhydride and 88.0 mL pyridine. The reaction mixture was stirred at 100°C for 135 min. The solvent was removed under reduced pressure. Toluene was added to the residue and the solvent was removed under reduced pressure, then 204 mL (816 mmol) HCl (4 M aqueous solution) was added and the mixture was refluxed for 3h. The solvent was removed under reduced pressure. 1-Butanol (20 mL) was added to the residue and the solvent was removed under reduced pressure. 11.6 g of the title compound were obtained as hydrochloride salt.
MS (ESI pos): m/z = 88 (M+H)⁺

### Example 3A (trans - racemic mixture)

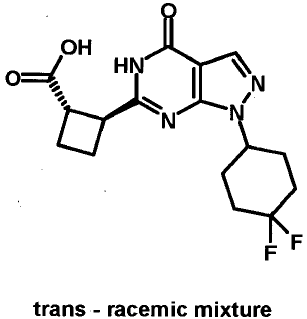

1.00 g (4.09 mmol) 5-Amino-1-(4,4-difluoro-cyclohexyl)-1H-pyrazole-4-carboxylic acid amide (see PCT patent application WO 2010/026214, example 8A) was mixed with 15 mL of anhydrous EtOH, 2.46 g (12.3 mmol) of Example 1A and 0.66 g (16.4 mmol) of sodium hydride (60 % suspension in mineral oil) were added. The reaction mixture was heated to 140°C for 30 min in a microwave oven. The mixture was cooled to room temperature and sodium hydroxide solution (4 M aqueous solution) was added. The solvent was removed under reduced pressure. The residue was purified by preparative HPLC (eluent A: water + 0.13 % TFA, eluent B: MeOH). 0.70 g (49 %) of the product were obtained.
HPLC-MS (Method 1): Rₜ = 1.24 min
MS (ESI pos): m/z = 353 (M+H)⁺

The following examples were synthesized in analogy to the preparation of Example 3 A, using the corresponding amide and ester as starting materials (for starting materials it is referred to PCT patent publications WO 2010/026214, WO 2009/121919 and WO 2004/09921).

| Example | structure | starting material: amide | starting material: ester | Rₜ [min] | MS (ESI pos, m/z) |
|---|---|---|---|---|---|
| Exp. 3B (trans - racemic mixture) | 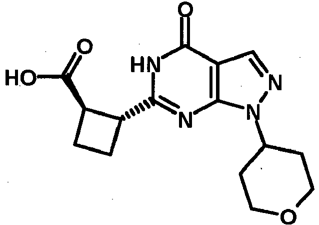 | 5-amino-1-(tetrahydropyran-4-yl)-1H-pyrazole-4-carboxylic acid amide (see WO 2009/121919, example 11B) | Exp. 1B | 1.07 (Method 3) | 319 (M+H)⁺ |
| Exp. 3C (trans-racemic mixture) | 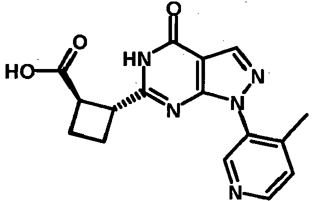 | 5-amino-1-(4-methyl-pyridin-3-yl)-1H-pyrazole-4-carboxylic acid amide (see WO 2004/099211, example 35A) | Exp. 1A | 0.81 (Method 1): | 326 (M+H)⁺ |

### Example 4A (trans - racemic mixture)

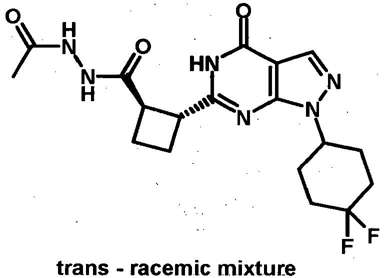

0.200 g (0.568 mmol) Example 3A were mixed with 0.157 mL (1.14 mmol) triethylamine and 5 mL DMF. To the mixture were added 0.237 g (0.624 mmol) HATU, then the reaction mixture was stirred at room temperature for 10 min. To the mixture were added 0.042 g (0.568 mmol) acetic acid hydrazide and the reaction mixture was stirred at room temperature for 1h. The mixture was purified by preparative HPLC (eluent A: water + 0.13 % TFA, eluent B: MeOH). 30 mg of the product were obtained.
HPLC-MS (Method 1): Rₜ = 1.03 min
MS (ESI pos): m/z = 409 (M+H)⁺

### Example 5A (trans - racemic mixture)

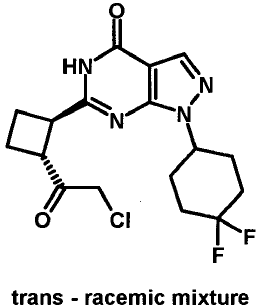

0.150 g (0.426 mmol) of Example 3A were mixed with 2 mL THF. The mixture was cooled to 0°C and 0.036 mL (0.426 mmol) oxalylchloride and one drop of DMF were added. The reaction mixture was stirred at 0°C for 1h. To the reaction mixture were added 2 mL ACN and 0.426 mL (0.851 mmol) trimethylsilyldiazomethane (2 M in hexane). The mixture was stirred for 2h, then 0.213 mL HCl (4 M in dioxane) was slowly added. The reaction was stirred for 3h. To the mixture were added ethylacetate and saturated aqueous sodium hydrogen carbonate solution. The organic layer was washed with water and brine and dried over sodium sulfate. The solvents were partially evaporated until volume of approximately 2 mL was reached. The mixture was taken to the next step without further purification.
HPLC-MS (Method 1): Rₜ = 1.40 min
MS (ESI pos): m/z = 385/387 (Cl)

The following example was synthesized in analogy to the preparation of Example 5A, using the corresponding acid as starting material.

| Example | structure | starting material | Rₜ [min] | MS (ESI pos, m/z) |
|---|---|---|---|---|
| Exp. 5B | 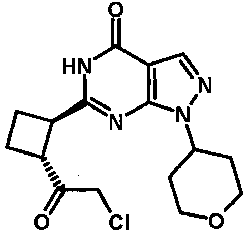 | Exp. 3B | 1.12 | 351/353 (Cl) |
| trans - racemic mixture | | | (Method 1) | |

### Example 6A (trans - mixture of stereoisomers)

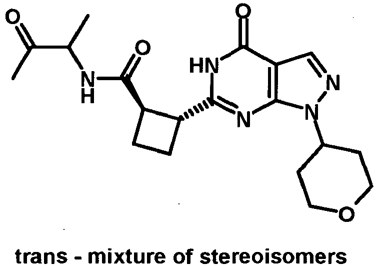

0.200 g (0.628 mmol) of Example 3B were mixed with 1 mL DMF. 0.261 mL (1.89 mmol) triethylamine and 0.222 g (0.691 mmol) of TBTU were added. The reaction mixture was stirred at room temperature for 10 min. Then 0.078 g (0.628 mmol) of Example 2A was added and the mixture was stirred at room temperature for 1h. The mixture was purified by preparative HPLC (eluent A: water + 0.13 % TFA, eluent B: MeOH). 190 mg of the product were obtained.
HPLC-MS (Method 3): Rₜ = 1.03 min
MS (ESI pos): m/z = 388 (M+H)⁺

### Example 7A (trans - racemic mixture)

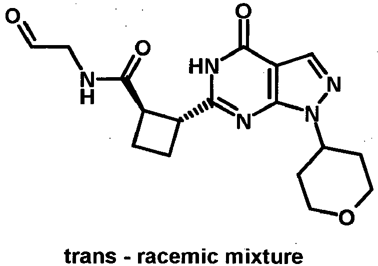

0.200 g (0.628 mmol) of Example 3B were mixed with 1 mL DMF. 0.174 mL (1.26 mmol) triethylamine and 0.222 g (0.691 mmol) of TBTU were added. The reaction mixture was stirred at room temperature for 10 min. Then 0.066 g (0.628 mmol) 2,2-dimethoxy-ethylamine was added and the mixture was stirred at room temperature for 1h. Then HCl (2 M aqueous solution) was added and the mixture was purified by preparative HPLC (eluent A: water + 0.13 % TFA, eluent B: MeOH). The residue was mixed with 5 mL acetone and 1 mL HCl (2 M aqueous solution) and stirred overnight under nitrogen. Then the mixture was extracted with DCM. The organic layer was evaporated and purified by preparative HPLC (eluent A: water + 0.13 % TFA, eluent B: MeOH). 170 mg of the product was obtained.
HPLC-MS (Method 3): Rₜ = 1.01 min
MS (ESI pos): m/z = 360 (M+H)⁺

### Example 8A trans - mixture of stereoisomers)

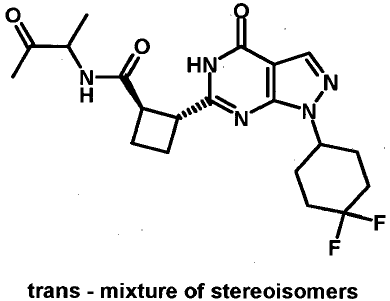

0.200 g (0.568 mmol) of Example 3A was mixed with 1.0 mL DMF. 0.432 mL (2.84 mmol) DIPEA and 0.200 g (0.624 mmol) TBTU were added. The reaction mixture was stirred at room temperature for 10 min. Then 0.140 g (1.14 mmol) of Example 2A were added and the mixture was stirred at room temperature for 2h. The mixture was purified by preparative HPLC (eluent A: water + 0.13 % TFA, eluent B: MeOH). 70 mg (29 %) of the product was obtained.
HPLC-MS (Method 1): Rₜ = 1.23 min
MS (ESI pos): m/z = 422 (M+H)⁺

The following examples were synthesized in analogy to the preparation of Example 8 A, using the corresponding nucleophiles as starting materials..

| Example | structure | starting material | Rₜ [min] | MS (ESI pos, m/z) |
|---|---|---|---|---|
| Exp. 8B | 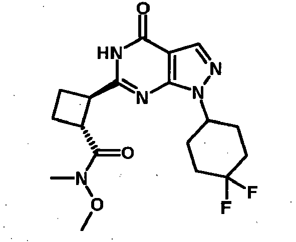 | 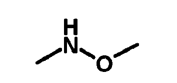 | 1.31 | 396 (M+H)⁺ |
| trans - racemic mixture | | hydrochloride | (method 1) | |
| Exp. 8C | 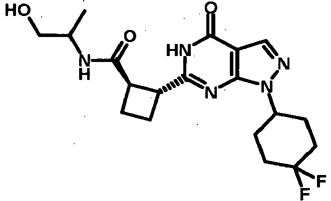 | 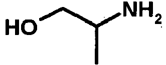 | | 410 (M+H)⁺ |
| trans - mixture of stereoisomers | | | | |
| Exp. 8D | 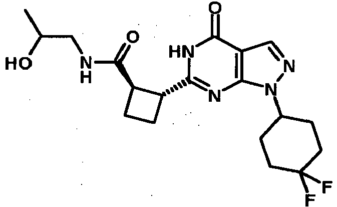 | 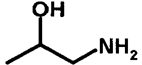 | 1.12 | 410 (M+H)⁺ |
| trans - mixture of stereoisomers | | | (method 1) | |
| Exp. 8E | 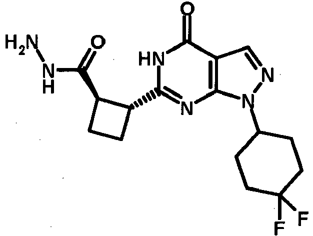 | hydrazine hydrate | 0.99 | 367 |
| trans - racemic mixture | | | (method 1) | (M+H)⁺ |

### Example 9A (trans - racemic mixture)

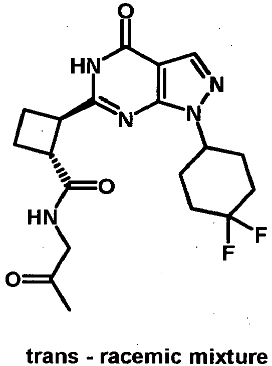

0.182 g (0.430 mmol) Dess-Martin periodinane were mixed with 2.5 mL DCM. 0.160 g (0.391 mmol) Example 8D in 2.5 mL DCM was added at room temperature. The reaction mixture stirred at room temperature for 30 min and at 30°C for 30 min. To the mixture were added 10 mL sodium thiosulfate solution (10 % in water) and 10 mL saturated sodium hydrogen carbonate solution and the mixture was stirred for 20 min. The organic layer was separated and the aqueous layer was extracted with DCM. The organic layer was washed with saturated sodium hydrogen carbonate solution, dried and evaporated. 93 mg (58 %) of the product were obtained.
HPLC-MS (Method 1): Rₜ = 1.18 min
MS (ESI pos): m/z = 408 (M+H)⁺

The following example was synthesized in analogy to the preparation of Example 9A, using the corresponding alcohol as starting material.

| Example | structure | starting material |
|---|---|---|
| Exp. 9B trans - mixture of stereoisomers | 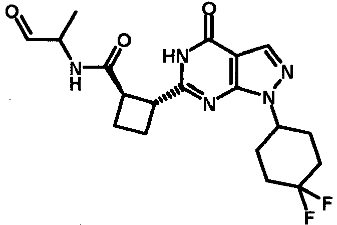 | Exp. 8C |

### Example 10A (trans - mixture of stereoisomers)

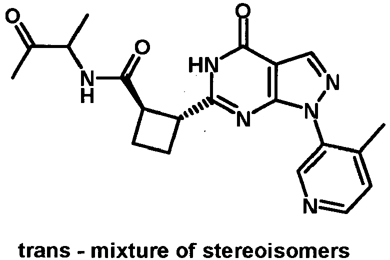

0.450 g of Example 3C was mixed with 3.5 mL DMF and 0.273 g (2.21 mmol) Example 2A. 1.00 mL (6.64 mmol) DIPEA and 0.390 g (1.22 mmol) TBTU were added and the mixture was stirred for 1h. The mixture was purified by preparative HPLC (eluent A: water + 0.13 % TFA, eluent B: MeOH). 360 mg (83 %) of the product was obtained.
HPLC-MS (Method 1): Rₜ = 0.85 min
MS (ESI pos): m/z = 395 (M+H)⁺

### Example 11A (trans - racemic mixture)

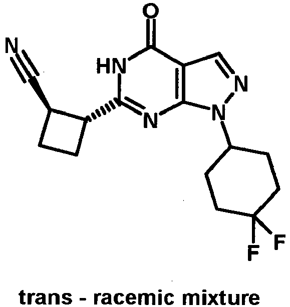

300 mg (1.23 mmol) of 5-amino-1-(4,4-difluoro-cyclohexyl)-1H-pyrazole-4-carboxylic acid amide (see WO 2010/026214, example 8A) were mixed with 4 mL anhydrous EtOH, 326 mg (3.07 mmol) trans-cyclobutane-1,2-dicarbonitrile and 0.197 g (4.91 mmol) of sodium hydride (60 % suspension in mineral oil) under nitrogen. The reaction mixture was heated to 140°C for 45 min in a microwave oven. The solvent was removed under reduced pressure. The residue was purified by preparative HPLC (eluent A: water + 0.13 % TFA, eluent B: MeOH). 210 mg (51 %) of the title compound were obtained.
HPLC-MS (Method 3): Rₜ = 1.19 min
MS (ESI pos): m/z = 334 (M+H)⁺

### Example 11B (trans - racemic mixture)

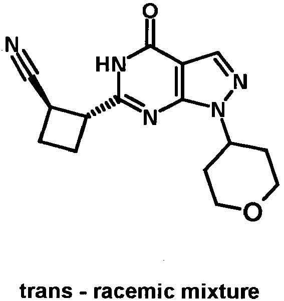

To a solution of 0.8 g (3.805 mmol) of 5-amino-1-(tetrahydro-pyran-4-yl)-1-H-pyrazole-4-carboxylic acid amide (see PCT patent application WO2010/026214) in 8 mL anhydrous EtOH , 0.457 g (19.6 mmol) of sodium hydride (60 % suspension in mineral oil) were added at room temperature under nitrogen. After 1 h under stirring, 1.2g (11.42mmol) of trans-cyclobutane-1,2-dicarbonitrile were added and the reaction mixture was heated to 140°C for 45 min in a microwave oven. The solvent was removed under reduced pressure. The residue was dissolved in DCM, water was added and phases were separated. Organic layers were dried over sodium sulphate and evaporated under reduced pressure. The crude was purified by flash cromatography (Cy/EtOAc from 80/20 to 100%) to obtain the title compound as yellow solid. (0.64g, 55%)
HPLC-MS(Method 1Eh):Rₜ=6.21 min
MS (APCI): m/z = 300 (M+H)⁺

### Example 11C (trans - racemic mixture)

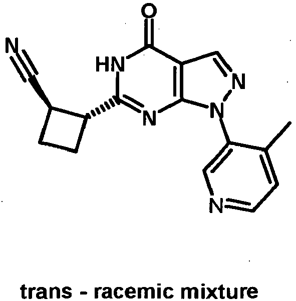

To a solution of 0.85 g (3.91 mmol) of 5-amino-1-(4-methyl-pyridin-3-yl)-1H-pyrazole-4-carboxylic acid amide (see PCT patent application WO 2004/09921) in 10 mL anhydrous EtOH , 0.47 g (11.74 mmol) of sodium hydride (60 % suspension in mineral oil) were added at room temperature under nitrogen. After 1 h under stirring, 1.28g (11.74 mmol) of trans-cyclobutane-1,2-dicarbonitrile were added and the reaction mixture was heated to 140°C for 45 min in a microwave oven. The reaction mixture was then loaded on SCX cartridge, ammonia fractions were collected and evaporated and the residue was purified by flash cromatography ( DCM/MeOH 90:10) to obtain the title compound as white solid. (0.63g, 52%).
HPLC-MS (Method 1Eh): Rₜ = 5.92 min
MS (APCI pos): m/z = 307 (M+H)⁺

### Example 12A (trans - racemic mixture)

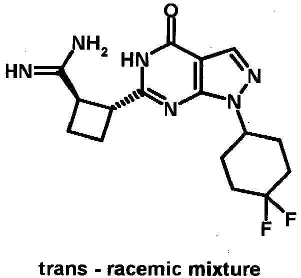

190 mg (0.570 mmol) of Example 11A were mixed with 0.281 mL toluene and 0.093 mL (2.30 mmol) anhydrous MeOH. 0.103 mL (1.45 mmol) acetylchloride were added slowly at 0°C. The mixture was stirred at room temperature for 12h. The solvent was removed under reduced pressure. To the residue 0.5 mL MeOH were added. Then 0.407 mL (2.85 mmol) ammonia (7 M in MeOH) were added at 0°C and the mixture was allowed to warm to room temperature. After 30 min the reaction mixture was treated with water and the pH was adjusted to pH=1 by addition of TFA. The mixture was purified by preparative HPLC (eluent A: water + 0.13 % TFA, eluent B: MeOH) yielding 110 mg (42 %) of the product were as trifluoroacetic acid salt.
HPLC-MS (Method 3): Rₜ = 1.04 min
MS (ESI pos): m/z = 351 (M+H)⁺

### Example 12 B (trans - racemic mixture)

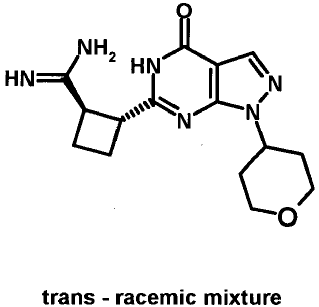

To a mixture of dry EtOH (5mL) and dry CHCl₃ (5mL) cooled at 0°C, acetylchloride (2.27mL, 30.82mmol) was added slowly and mixture left under stirring for 20min. 0°C. A solution of Example 11B (0.410g, 1.027mmol) in dry CHCl₃ (5mL) was added dropwise and the mixture stirred at room temperature overnight. Solvents were evaporated under reduced pressure, residue dissolved in dry EtOH (5mL) and 6.4mL of a 7.0M solution of ammonia in MeOH (30.82mmol) were added. The mixture was stirred at room temperature for 12h. The solvent was removed under reduced pressure. The final product was obtained as hydrochloride and used for the next step without further purification. (0.37g, content 50% estimated by HPLC-MS).
HPLC-MS (Method 1Eh): Rₜ = 5.38 min
MS (APCI pos): m/z = 317 (M+H)⁺

### Example 12 C (trans - racemic mixture)

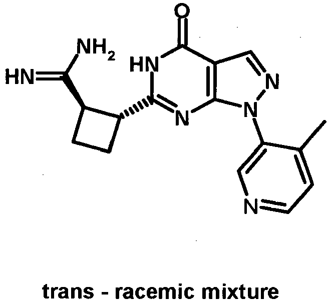

To a mixture of dry EtOH (4mL) and dry CHCl₃ (10mL cooled at 0°C, acetylchloride (4.38 mL, 61.7 mmol) was added slowly and mixture left under stirring for 20min. 0°C. A solution of Example 11C (0.63g, 2.057 mmol) in dry CHCl₃ (5mL) was added dropwise and the mixture stirred at room temperature overnight. Solvents were evaporated under reduced pressure, residue dissolved in dry MeOH (10mL) and 10.3 mL of a 7.0M solution of ammonia in MeOH (72 mmol) were added. The mixture was stirred at room temperature for 12h. The solvent was removed under reduced pressure. The final product, obtained as hydrochloride salt, was used as such in the next step without further purification. (0.85g, content 84%, estimated by 1H-NMR).
HPLC-MS (Method 1Eh): Rₜ = 5.15 min
MS (APCI pos): m/z = 324 (M+H)⁺

### Example 13A (trans - racemic mixture)

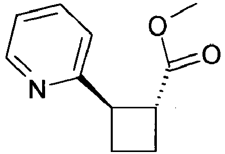

To a solution of 1.6 g (10.24 mmol) of 2-acetyl-cyclobutanecarboxylic acid methyl ester (prepared as described in J. Med. Chem, 25, 109, 1982) in dry EtOH (12mL), propargylamine (1.4mL, 20.4mmmol) was added followed by 0.122g (0.307mmol) of sodium gold trichloride. The reaction mixture was heated to 140°C for 45 min in a microwave oven, solid was filtered and the organic evaporated. Crude was purified by flash cromatography (Cy/EtOAc 70:30) to obtain the title compound as yellow green oil. (0.18g, 9.2%).
HPLC-MS (Method 1Eh): Rₜ = 0.87 min
MS (APCI pos): m/z = 192 (M+H)⁺

### Exemplary embodiments

### Example 1 (trans - racemic mixture)

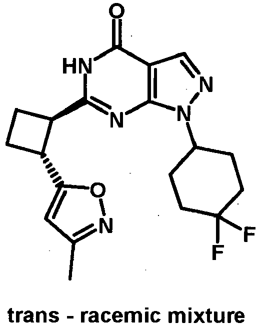

22.0 mg (0.306 mmol) of propan-2-one oxime were mixed with 2 mL anhydrous THF and 0.471 mL (1.22 mmol) n-butyllithium (2.6 mol/L in toluene) was added carefully to the mixture. The reaction mixture was stirred at room temperature for 30 min. 0.110 g (0.278 mmol) of Example 8B in 1 mL anhydrous THF were carefully added during 10 min. After 30 min the reaction mixture was added to a mixture of 0.28 mL H₂SO₄ and 4 mL THF/water (4:1). The mixture was refluxed for 1.5h. Saturated aqueous sodium hydrogen carbonate solution was added and extracted with ethylacetate. The organic layer was dried and the solvents were evaporated. The residue was purified by preparative HPLC (eluent A: water + 0.13 % TFA, eluent B: MeOH). 8 mg (8 %) of the product were obtained.
HPLC-MS (Method 1): Rₜ = 1.40 min
MS (ESI pos): m/z = 390 (M+H)⁺

### Example 2 (trans - racemic mixture)

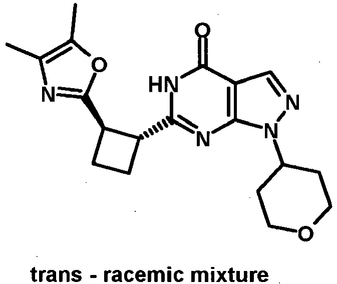

0.190 g of Example 6A were mixed with 3 mL DME and 0.273 g (1.14 mmol) Burgess reagent. The reaction mixture was heated to 130°C for 1h in a microwave oven. The solvent was evaporated and the residue purified by preparative HPLC (eluent A: water + 0.13 % TFA, eluent B: MeOH). 70 mg (55 %) of the product were obtained.
HPLC-MS (Method 1): Rₜ = 1.11 min
MS (ESI pos): m/z = 370 (M+H)⁺

The following examples were synthesized in analogy to the preparation of Example 2, using the corresponding amides as starting materials.

| Example | structure | starting material | Rₜ [min] | MS (ESI pos, m/z) |
|---|---|---|---|---|
| Exp. 3 trans - racemic mixture | 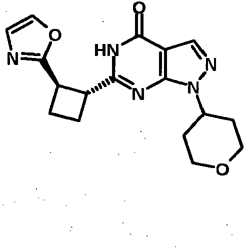 | Exp. 7A | 1.17 (Method 3) | 342 (M+H)⁺ |
| Exp. 4 trans - racemic mixture | 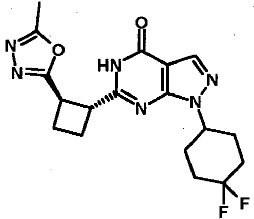 | Exp. 4A | 1.20 (Method 1) | 391 (M+H)⁺ |
| Exp. 5 trans - racemic mixture | 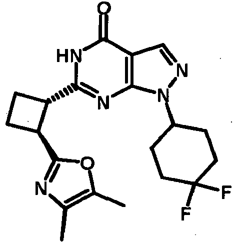 | Exp. 8A | 1.38 (method 1) | 404 (M+H)⁺ |
| Exp. 6 trans - racemic mixture | 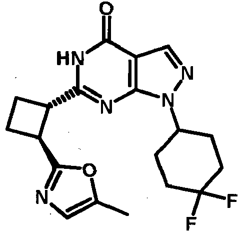 | Exp. 9A | 1.37 (method 1) | 390 (M+H)⁺ |
| Exp. 7 trans - racemic mixture | 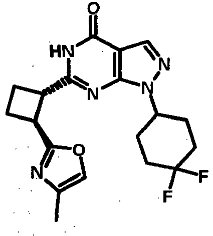 | Exp. 9B | 1.42 (method 3) | 390 (M+H)⁺ |
| Exp. 8 trans - racemic mixture | 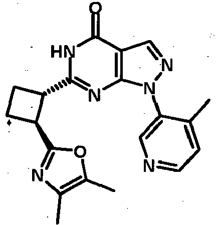 | Exp. 10A | 0.97 (method 1) | 377 (M+H)⁺ |

### Example 9 (trans - racemic mixture)

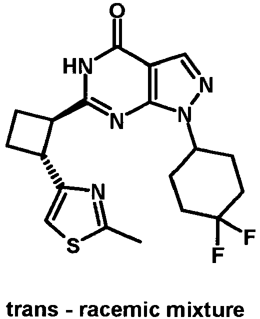

To a solution of Example 5A, synthesized starting from 0.426 mmol of Example 3A as described above, was added dropwise 0.062 g (0.832 mmol) thioacetamide in 2 mL EtOH. The reaction mixture was stirred overnight. The mixture was purified by preparative HPLC (eluent A: water + 0.13 % TFA, eluent B: MeOH). 62 mg of the title compound were obtained.
HPLC-MS (Method 1): Rₜ = 1.37 min
MS (ESI pos): m/z = 406 (M+H)⁺

The following examples were synthesized in analogy to the preparation of Example 9, using the corresponding starting materials.

| Example | structure | starting material: nucleophile | starting material: chloroketon | Rₜ [min] | MS (ESI pos, m/z) |
|---|---|---|---|---|---|
| Exp. 10 trans - racemic mixture | 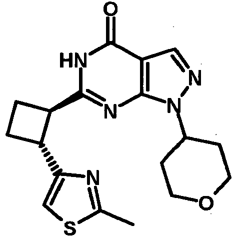 | thioacetamide | Exp. 5B | 1.21 (Method 3) | 372 (M+H)⁺ |
| Exp. 11 trans - racemic mixture | 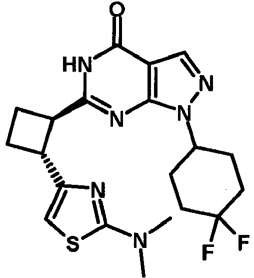 | 1,1-dimethylthiourea | Exp. 5A | 1.15 (Method 3) | 435 + (M+H) |
| Exp. 12 trans - racemic mixture | 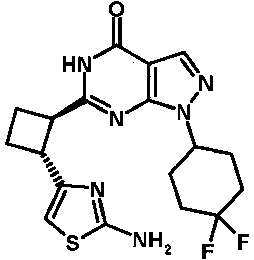 | thiourea | Exp. 5A | 1.15 (Method 3) | 407 (M+H)⁺ |

### Example 13 (trans - racemic mixture)

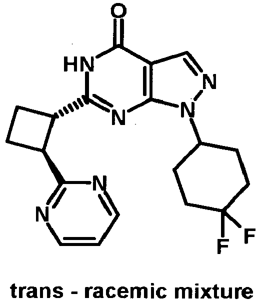

100 mg (0.215 mmol) of Example 12A were mixed with 1.00 mL (6.07 mmol) 1,1,3,3-tetramethoxypropane. The reaction mixture was heated to 175°C for 1h using a microwave oven. The reaction mixture was treated with DCM/MeOH and one drop of triethylamine. The solvents were removed under reduced pressure. The mixture was purified by preparative HPLC (eluent A: water + 0.13 % TFA, eluent B: MeOH) yielding 45 mg (54 %) of the title compound.
HPLC-MS (Method 3): Rₜ = 1.36 min
MS (ESI pos): m/z = 387 (M+H)⁺

The enantiomers of the title compound were separated by HPLC using a chiral stationary phase.

### Method for enantioseparation:

HPLC apparatus type: Berger Minigram; column: Daicel IC, 5.0 µm, 250 mm x 10 mm; method: eluent CO₂ / 30 % MeOH / 0.2 % DEA (isocratic); flow rate: 10 mL/min, Temperature: 40°C; pressure: 100 bar; UV Detection: 210 nm

| Example | structure | Rₜ [min] |
|---|---|---|
| Exp. 14 trans - enantiomer 1 | 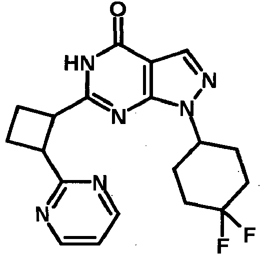 | 3.15 (Method 4) |
| | | |
| Exp. 15 trans - enantiomer 2 | 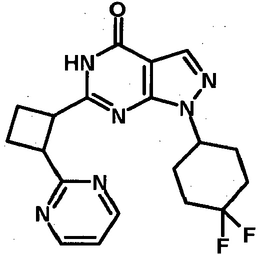 | 3.78 (Method 4) |

The following example was synthesized in analogy to the preparation of Example 13, using the corresponding dialdehydediacetal as starting material.

| Example | structure | starting material | Rₜ [min] | MS (ESI pos, m/z) |
|---|---|---|---|---|
| Exp. 16 trans - racemic mixture | 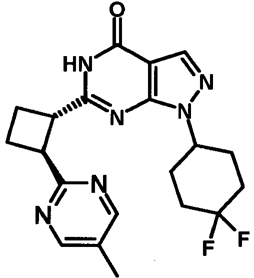 | 1,1,3,3-tetraethoxy-2-methylpropane | 1.42 (Method 3) | 401 (M+H)⁺ |

### Example 17 (trans - racemic mixture)

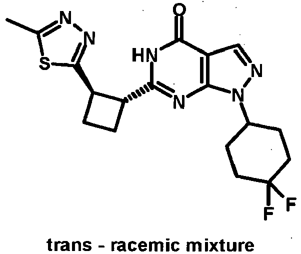

176 mg (0.431 mmol) of Example 4A were mixed with 3 mL THF and 122 mg (0.302 mmol) Lawesson's reagent at room temperature. Then the mixture was stirred for 6h at 60°C. The reaction mixture was treated with water and diluted with DCM. The mixture was filtered over basic alumia and eluted with DCM und EtOH. The solvents were removed under reduced pressure. The residue was purified by preparative HPLC (eluent A: water + 0.13 % TFA, eluent B: MeOH). 45 mg (26 %) of the product were obtained.
HPLC-MS (Method 3): Rₜ = 1.37 min
MS (ESI pos): m/z = 407 (M+H)⁺

The enantiomers of the title compound were seperated by HPLC using a chiral stationary phase.

### Method for enantioseparation:

HPLC apparatus type: Berger Minigram; column: Daicel ADH, 5.0 µm, 250 mm x 10 mm; method: eluent CO₂ / 30 % MeOH / 0.2 % DEA (isocratic); flow rate: 10 mL/min, Temperature: 40°C; pressure: 100 bar; UV Detection: 210 nm

| Example | structure | Rₜ [min] |
|---|---|---|
| Exp. 18 trans - enantiomer 1 (S,S) | 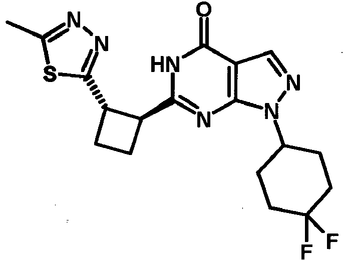 | 2.47 (Method 5) |
| Exp. 19 trans - enantiomer 2 (R,R) | 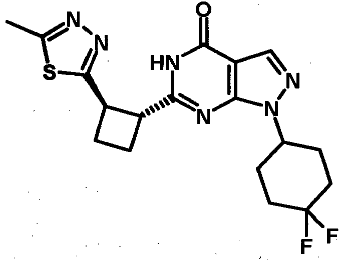 | 2.96 (Method 5) |

Single crystals of example 19 have been prepared by recrystallisation from ethylacetate and subjected to X-ray crystal analysis. The data allowed to determine the absolute configuration of example 19 to be (R,R).
Experimental: Data collection and reduction: Data collected on Saturn 944 CCD mounted on AFC11K goniometer, Radiation: Cu Kα from RU200 rotating anode and RIGAKU VARIMAX optics, Temperature: 100K.

### Summary of data collection statistics

| | | |
|---|---|---|
| Spacegroup | P2₁ | |
| Unit cell dimensions | 8.560(2) 6.844(1) 15.603(3) 90.00 98.82(3) 90.00 | |
| Resolution range | 15.42 - 0.85 (0.88 - 0.85) | |
| Total number of reflections | 10857 | |
| Number of unique reflections | 1588 | |
| Average redundancy | 6.84 | (2.46) |
| % completeness | 95.7 | (79.1) |
| Rmerge | 0.064 | (0.118) |
| Output <I/sigI> | 27.7 | (7.9) |

Values in () are for the last resolution shell.

### Refinement statistics:

Final Structure Factor Calculation for example 19 in P2₁
Total number of 1.s. parameters = 255
GooF = S = 1.154
Weight = 1 / [sigma^2(Fo^2) + (0.0421 * P)^2 + 0.38 * P] where P = (Max (Fo^2, 0) + 2*Fc^2)/3
R1 = 0.0695 for 2207 Fo > 4sig(Fo)and 0.0829 for all 2334 data, wR2 = 0.1646,
Flack x parameter = 0.09(3).

### Example 20 (trans - racemic mixture)

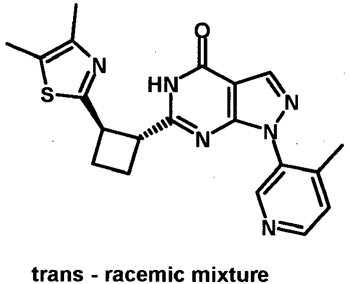

0.060 g of Example 10A were mixed with 4 mL anhydrous dioxane and 0.074 g (0.180 mmol) Lawesson's reagent. The reaction mixture was heated to 120°C for 1h in a microwave oven. The mixture was filtered over basic alumina and eluted with DCM and MeOH. The solvents were removed under reduced pressure. The residue was purified by preparative HPLC (eluent A: water + 0.13 % TFA, eluent B: MeOH). 22 mg of the product were obtained as salt with TFA.
HPLC-MS: (Method 1): Rₜ = 0.94 min
MS (ESI pos): m/z = 393 (M+H)⁺

### Example 21 (trans - racemic mixture)

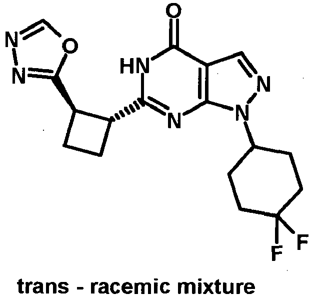

0.190 g (0.519 mmol) Example 8E were mixed with 1.38 mL (8.31 mmol) triethoxymethane. The mixture was stirred for 1.5h at 150°C. The reaction mixture was allowed to cool to room temperature and purified by preparative HPLC (eluent A: water + 0.13 % TFA, eluent B: MeOH). 90 mg (46 %) of the product were obtained.
HPLC-MS (Method 1): Rₜ = 1.19 min
MS (ESI pos): m/z = 377 (M+H)⁺

### Example 22 (trans - racemic mixture)

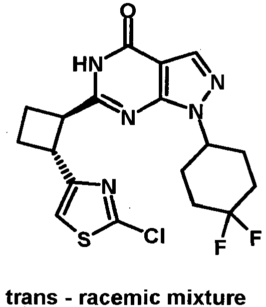

13 mg (0.10 mmol) CuCl₂, 26 mL (0.22 mmol) tert-butyl-nitrite were mixed with ACN. A mixture of 22 mg (0.05 mmol) Example 12 in ACN was carefully added at 0°C. The mixture was stirred for 1h at 25°C. Additional 9 mg (0.07 mmol) CuCl₂ and 13 mL (0.11 mmol) tert-butyl-nitrite was added and stirred another 20 min. The solvents were removed under reduced pressure. The residue was taken up in DCM and extracted with HCl and water. The mixture was purified by preparative HPLC (eluent A: water + 0.13 % TFA, eluent B: MeOH) yielding2.1 mg (9 %) of the product..
HPLC-MS: (Method 3): Rₜ = 1.46 min
MS (ESI pos): m/z = 426/428 (Cl) (M+H)⁺

### Example 23 (trans - racemic mixture)

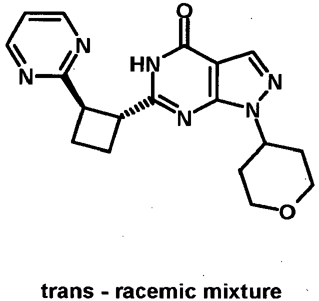

180 mg (0.26 mmol, content 50%, estimated by HPLC-MS) of Example 12b were mixed with 1.00 mL (6.07 mmol) 1,1,3,3-tetramethoxypropane. The reaction mixture was heated to 175°C for 1h using a microwave oven. The reaction mixture was treated with DCM, washed with water. Organic layers were dried over sodiumsulphate and evaporated under reduced pressure. The crude was purified by flash cromatography (Cy/EtOAc from 80/20 to AcOEt/MeOH 96/4) and then with a second flash cromatography (DCM 100% to DCM/EtOH 96/4) to obtain the title compound as beige solid. (0.034g).
HPLC-MS (Method 1Eh): Rₜ = 6.57 min
MS (APCI pos): m/z = 353 (M+H)⁺

The enantiomers of the title compound were seperated by HPLC using a chiral stationary phase.

### Method for enantioseparation:

### Semipreparative conditions:

HPLC semipreparative system: Waters 600 pump; column: Daicel chiralcel OJ-H, 250 mm x 20 mm, 5.0 µm; eluent: hexane/EtOH80:20; flow rate: 15 mL/min, Temperature: 25°C; UV Detection: 254 nm

| Example | structure | Rₜ [min] |
|---|---|---|
| Exp. 24 trans - enantiomer 1 | 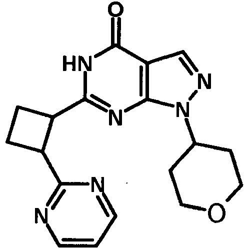 | 15.604 (Method 6) |
| Exp. 25 trans - enantiomer 2 | 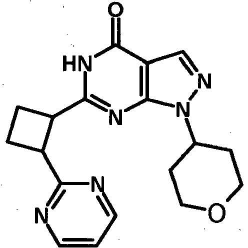 | 20.119 (Method 6) |

### Analytical conditions

HPLC apparatus type: Agilent 1100; Method 6; column: Daicel chiralcel OJ-H, 250 mm x 4.6 mm, 5.0 µm; eluent: hexane/EtOH80:20; flow rate: 1 mL/min, Temperature: 25°C; UV Detection: 254 nm

### Example 26 trans - racemic mixture)

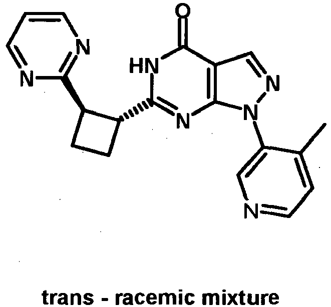

140 mg (content 84%, 0.33 mmol) of Example 12C were mixed with 1.4 mL of 1,1,3,3-tetramethoxypropane and 1.4mL of NMP. The reaction mixture was heated to 175°C for 1h using a microwave oven. The reaction mixture was then diluted with MeOH and loaded on SCX cartridge. Ammonia fractions were collected and the residue was purified by flash cromatography (Cy/EtOAc from 90/10 to 100%) to obtain the title compound as white solid (30mg).
HPLC-MS (Method 1Eh): Rₜ = 6.72 min
MS (APCIpos): m/z = 370 (M+H)⁺

The enantiomers of the title compound were seperated by HPLC using a chiral stationary phase.

### Method for enantioseparation:

### Semipreprative conditions:

HPLC semipreparative system: Waters 600 pump; column: Daicel chiralcel OJ-H, 250 mm x 20 mm, 5.0 µm; eluent: hexane/EtOH80:20; flow rate: 15 mL/min, Temperature: 25°C; UV Detection: 230 nm

| Example | structure | Rₜ [min] |
|---|---|---|
| Exp. 27 trans - enantiomer 1 | 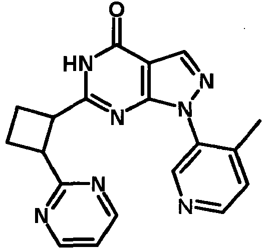 | 17.748 (Method 6) |
| Exp. 28 trans - enantiomer 2 | 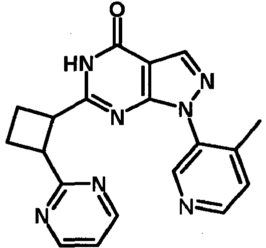 | 20.475 (Method 6) |

### Analytical conditions

HPLC apparatus type: Agilent 1100; Method 6; column: Daicel chiralcel OJ-H, 250 mm x 4.6 mm, 5.0 µm; eluent: hexane/EtOH80:20; flow rate: 1 mL/min, Temperature: 25°C; UV Detection: 254 nm

### Example 29 (trans - racemic mixture)

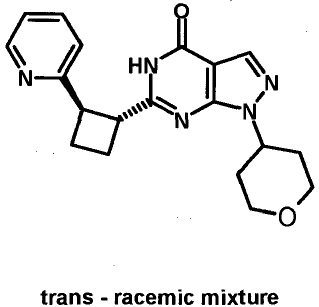

To a suspension of 0.132 g (0.63mmol) of 5-amino-1-(tetrahydro-pyran-4-yl)-1-H-pyrazole-4-carboxylic acid amide (see PCT patent application WO2010/026214) in dry EtOH (1.5mL), 0.066 g (1.66 mmol) of sodium hydride (60 % suspension in mineral oil) were added at room temperature under nitrogen. After 10min, 0.181mg (0.945mmol) of Example 13A were added and the reaction mixture was heated to 140°C for 40 min in a microwave oven (Power 100W). The reaction mixture was then diluted with DCM, water was added, organics separated and dried over sodiumsulphate. Organics were evaporated under reduced pressure and the crude purified by flash cromatography (DCM/IPA 98:2) to obtain the title compound as a white solid. (54mg, 32%).
HPLC-MS (Method 1Eh): Rₜ = 8.01 min
MS (APCI pos): m/z = 352 (M+H)⁺

The enantiomers of the title compound were seperated by HPLC using a chiral stationary phase.

### Method for enantioseparation:

### Semipreprative conditions:

HPLC semipreparative system: Waters 600 pump; column: Daicel chiralcel OJ-H, 250 mm x 20 mm, 5.0 µm; eluent: hexane/EtOH85:15; flow rate: 15 mL/min, Temperature: 25°C; UV Detection: 254 nm

| Example | structure | Rₜ [min] |
|---|---|---|
| Exp. 30 trans - enantiomer 1 | 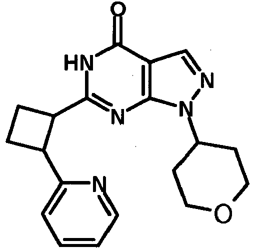 | 14.754 (Method 6.1) |
| Exp. 31 trans - enantiomer 2 | 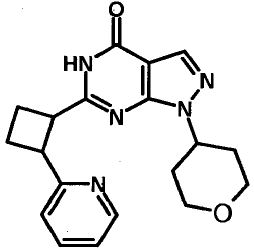 | 16.834 (Method 6.1) |

### Analytical conditions

HPLC apparatus type: Agilent 1100; Method 6.1; column: Daicel chiralcel OJ-H, 250 mm x 4.6 mm, 5.0 µm; eluent: hexane/EtOH85:15; flow rate: 1 mL/min, Temperature: 25°C; UV Detection: 254 nm

### Example 32 (trans - racemic mixture)

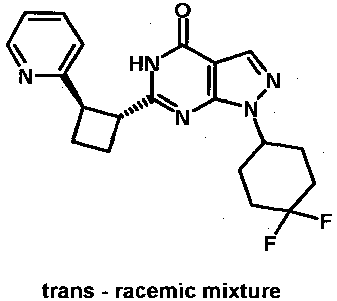

To a suspension of 0.135 g (0.553 mmol) of 5-amino-1-(4,4-difluoro-cyclohexyl)-1-H-pyrazole-4-carboxylic acid amide (see PCT patent application WO2010/026214) in dry EtOH (1.5mL), 0.066 g (1.66 mmol) of sodium hydride (60 % suspension in mineral oil) were added at room temperature under nitrogen. After 10min, 0.161mg (0.837mmol) of Example 13A were added and the reaction mixture was heated to 140°C for 40 min in a microwave oven (Power 100W). The reaction mixture was then diluted with DCM, water was added, organics separated and dried over sodium sulphate. Organics were evaporated under reduced pressure and the crude purified by flash cromatography (Cy/EA from 50:50 to 10:90) to obtain the title compound as a white solid. (54mg, 25%).
HPLC-MS (Method 1Eh): Rₜ = 9.63 min
MS (APCI pos): m/z = 386 (M+H)⁺

The enantiomers of the title compound were seperated by HPLC using a chiral stationary phase.

### Method for enantioseparation:

### Semipreprative conditions:

HPLC semipreparative system: Waters 600 pump; Column: Daicel chiralpak AD-H, 250 mm x 20 mm, 5.0 µm; eluent: hexane/Isopropanol 80:20; flow rate: 10 mL/min, Temperature: 25°C; UV Detection: 260 nm

| Example | structure | Rₜ [min] |
|---|---|---|
| Exp. 33 trans - enantiomer 1 | 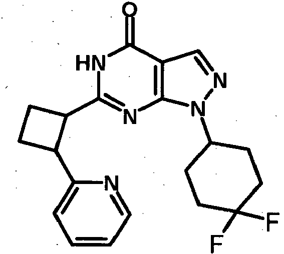 | 14.80 (Method 7) |
| Exp. 34 trans - enantiomer 2 | 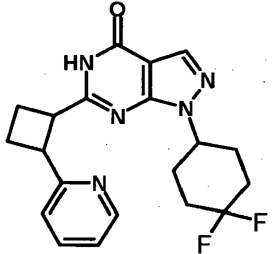 | 20.40 (Method 7) |

### Analytical conditions

HPLC apparatus type: Agilent 1100; Method 7; column: Daicel chiralcel AD-H, 250 mm x 4.6 mm, 5.0 µm; eluent: hexane/Isopropanol 80:20; flow rate: 1 mL/min, Temperature: 25°C; UV Detection: 260 nm.

## Claims

1. A compound of formula (I) 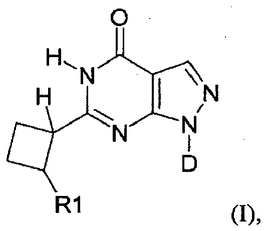 wherein the compound is selected from the group of 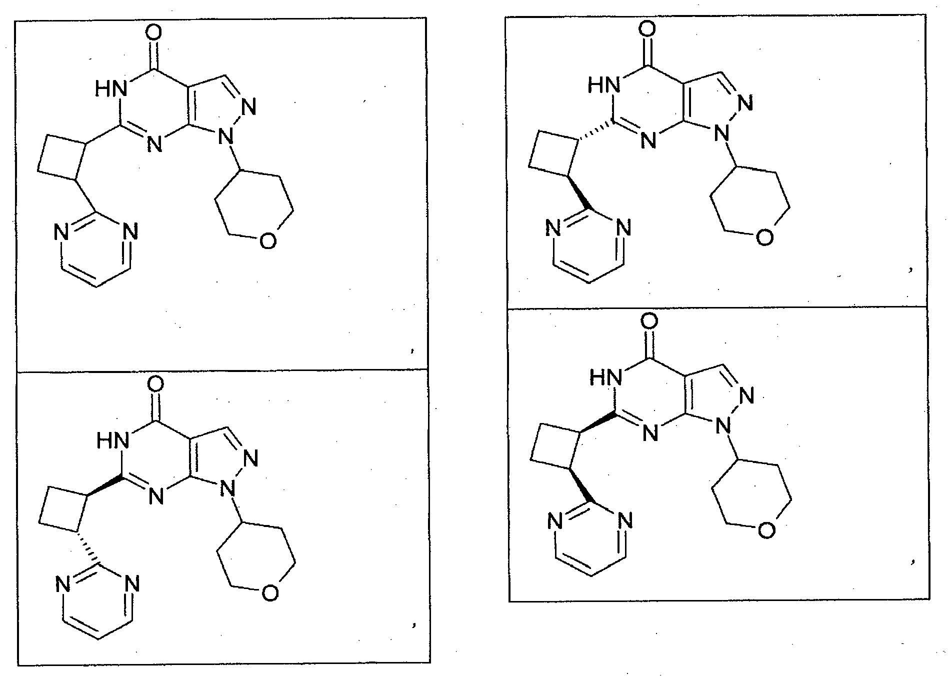 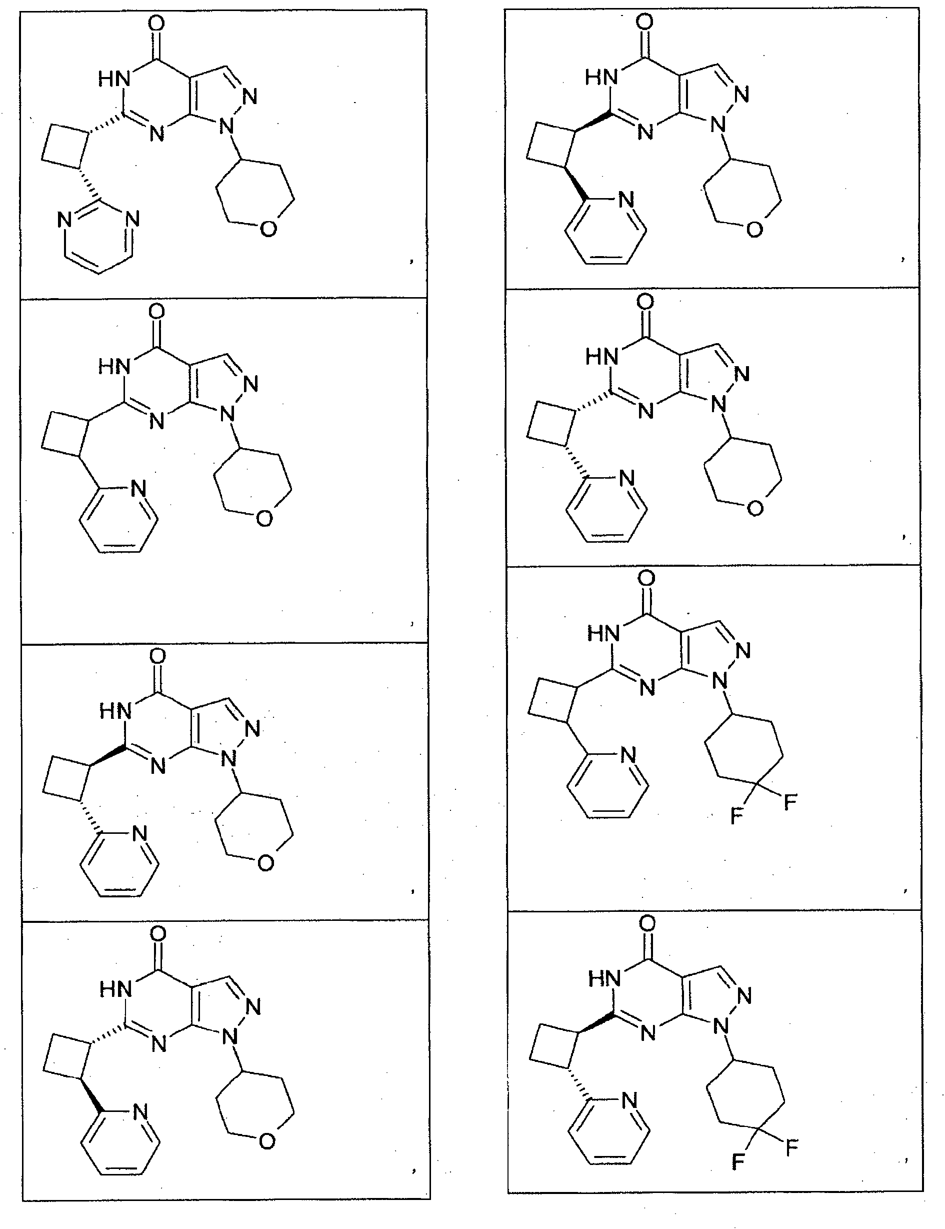 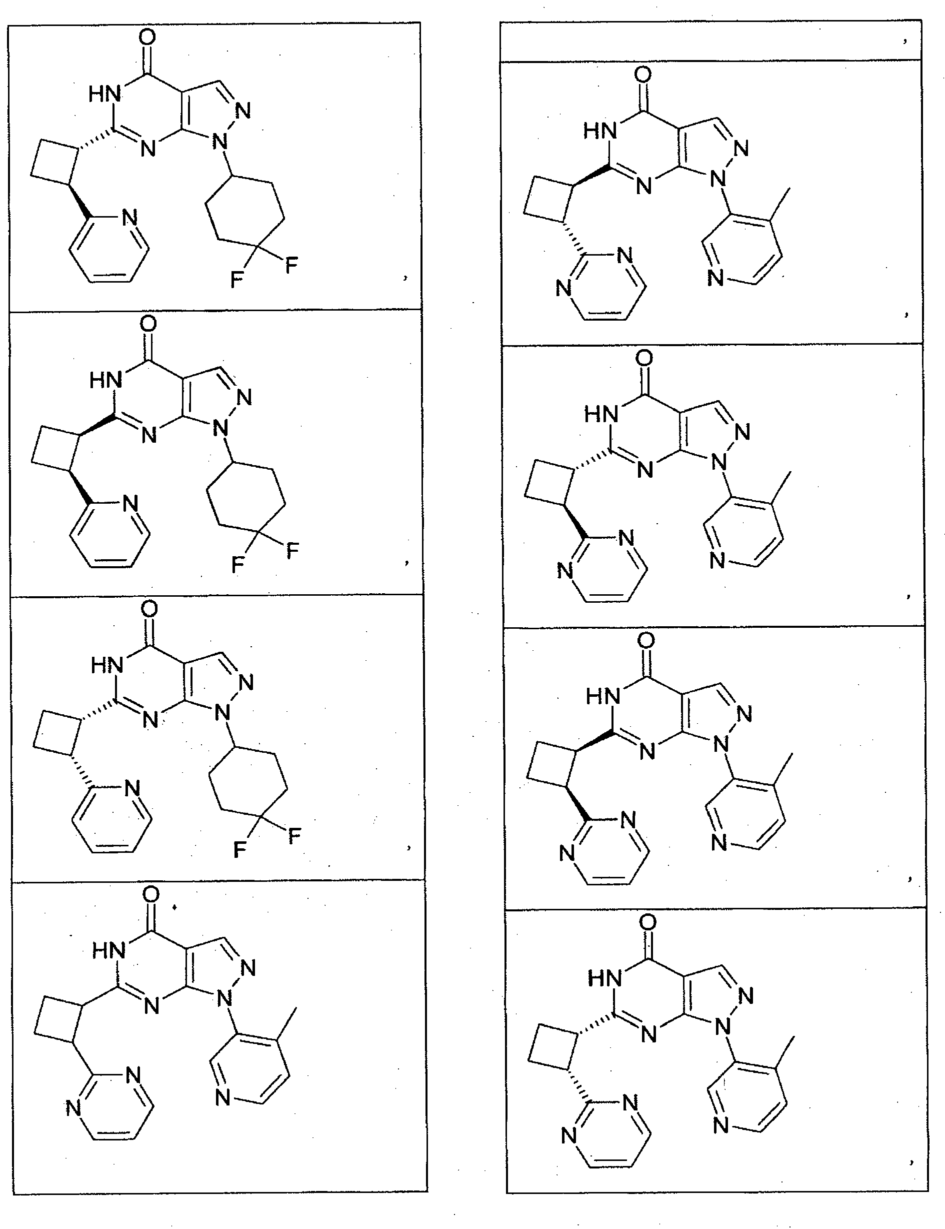 and salts, preferably pharmaceutically acceptable salts thereof.

2. The compound according to claim 1, whereby the compound is selected from the group of a compound according to formula (IIa). 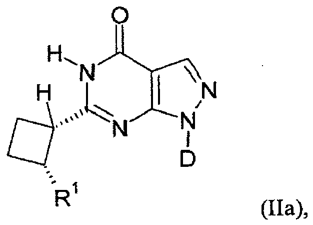 and salts, preferably pharmaceutically acceptable salts thereof.

3. The compound according to claim 1, whereby the compound is selected from the group of a compound according to formula (IIb). 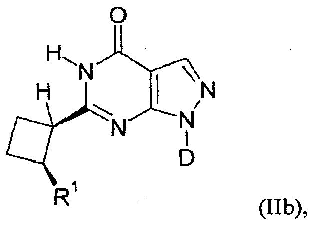 and salts, preferably pharmaceutically acceptable salts thereof.

4. The compound according to claim 1, whereby the compound is selected from the group of a compound according to formula (IIc). 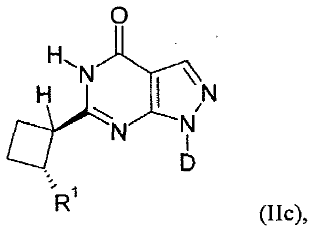 and salts, preferably pharmaceutically acceptable salts thereof.

5. The compound according to claim 1, whereby the compound is selected from the group of a compound according to formula (IId). 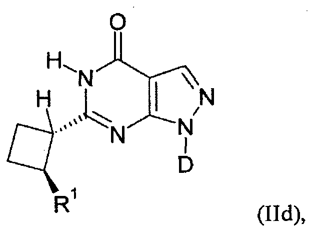 and salts, preferably pharmaceutically acceptable salts thereof.

6. A compound according to any one of claims 1 to 5 for use as a medicament or for the use as pharmaceutically active ingredient in a medicament, preferably a medicament for use in a therapeutic or prophylactic method, preferably in a therapeutic method.

7. A compound according to any one of claims 1 to 5 for use in
(a) the treatment of a CNS disease, more preferably a CNS disease, the treatment of which is accessible by the inhibition of PDE9,
(b) the treatment of a disease that is accessible by the inhibition of PDE9,
(c) the treatment of or for the amelioration of or for the prevention of, preferably for the treatment of a condition being selected from the group consisting of cognitive impairment being related to a disease or condition selected from the group of perception, concentration, cognition, learning or memory, whereby preferably the treatment, amelioration or prevention of cognitive impairment is related to age-associated learning and memory impairments, age-associated memory losses, vascular dementia, craniocerebral trauma, stroke, dementia occurring after strokes (post stroke dementia), post-traumatic dementia, general concentration impairments, concentration impairments in children with learning and memory problems, Alzheimer's disease, Lewy body dementia, dementia with degeneration of the frontal lobes, including Pick's syndrome, Parkinson's disease, progressive nuclear palsy, dementia with corticobasal degeneration, amyotropic lateral sclerosis (ALS), Huntington's disease, multiple sclerosis, thalamic degeneration, Creutzfeld-Jacob dementia, HIV dementia, epilepsy, temporal lobe epilepsy, schizophrenia, schizophrenia (with dementia), Korsakoffs psychosis or cognitive impairment associated with depression or bipolar disorder,
(d) the treatment of Alzheimer's disease or cognitive impairment associated with Alzheimer's disease,
(e) the treatment of schizophrenia or cognitive impairment associated with schizophrenia,
(f) the treatment of epilepsy or cognitive impairment associated with epilepsy,
(g) the treatment of a disease or condition being selected from the group consisting of sleep disorders, bipolar disorder, metabolic syndrome, obesity, diabetes mellitus, hyperglycemia, dyslipidemia, impaired glucose tolerance, or a disease of the testes, brain, small intestine, skeletal muscle, heart, lung, thymus or spleen.

8. A compound according to any one of claims 1 to 5 for use in the treatment or prophylaxis of cognitive impairment, preferable cognitive impairment related to perception, concentration, learning or memory.

9. A compound according to any one of claims 1 to 5 for use in the treatment or prophylaxis of cognitive impairment, preferable cognitive impairment related to perception, concentration, learning or memory, as symptoms of anther base laying disease.

10. A compound according to any one of claims 1 to 5 for use in the improvement of cognitive skills related to perception, concentration, learning or memory.

11. Pharmaceutical composition comprising a compound according to any one of claims 1 to 5 and a pharmaceutical carrier.

12. Combination of a compound according to any one of claims 1 to 5 with another active agent, whereby
- said combination preferably is useful for the treatment of a disease or condition as defined in any of claims 7 to 10 or
- said combination preferably being for the use in a therapeutic or prophylactic method, preferably a therapeutic method, for the treatment of a condition or disease as defined in any of claims 7 to 10 or
- said combination being for the manufacture of a medicament which preferably is for the treatment of a condition or disease as defined in any of claims 7 to 10.

## Patentansprüche

1. Verbindung der Formel (I) 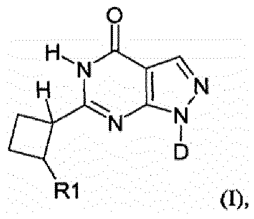 wobei die Verbindung ausgewählt ist aus der Gruppe 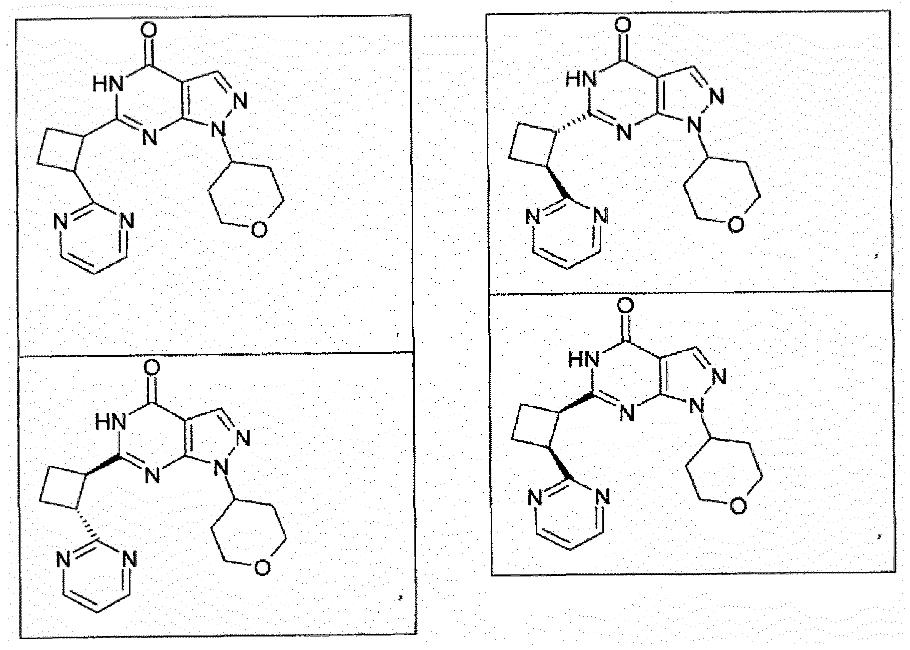 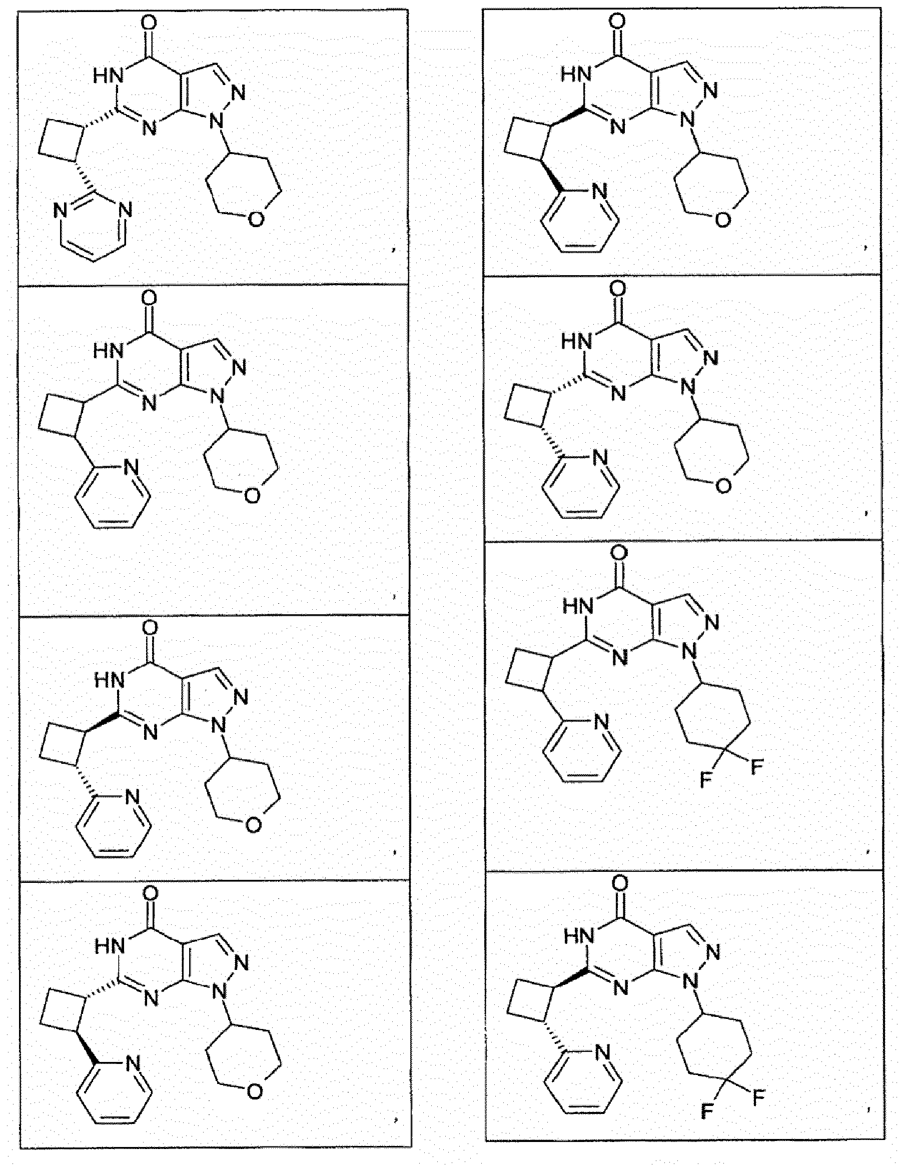 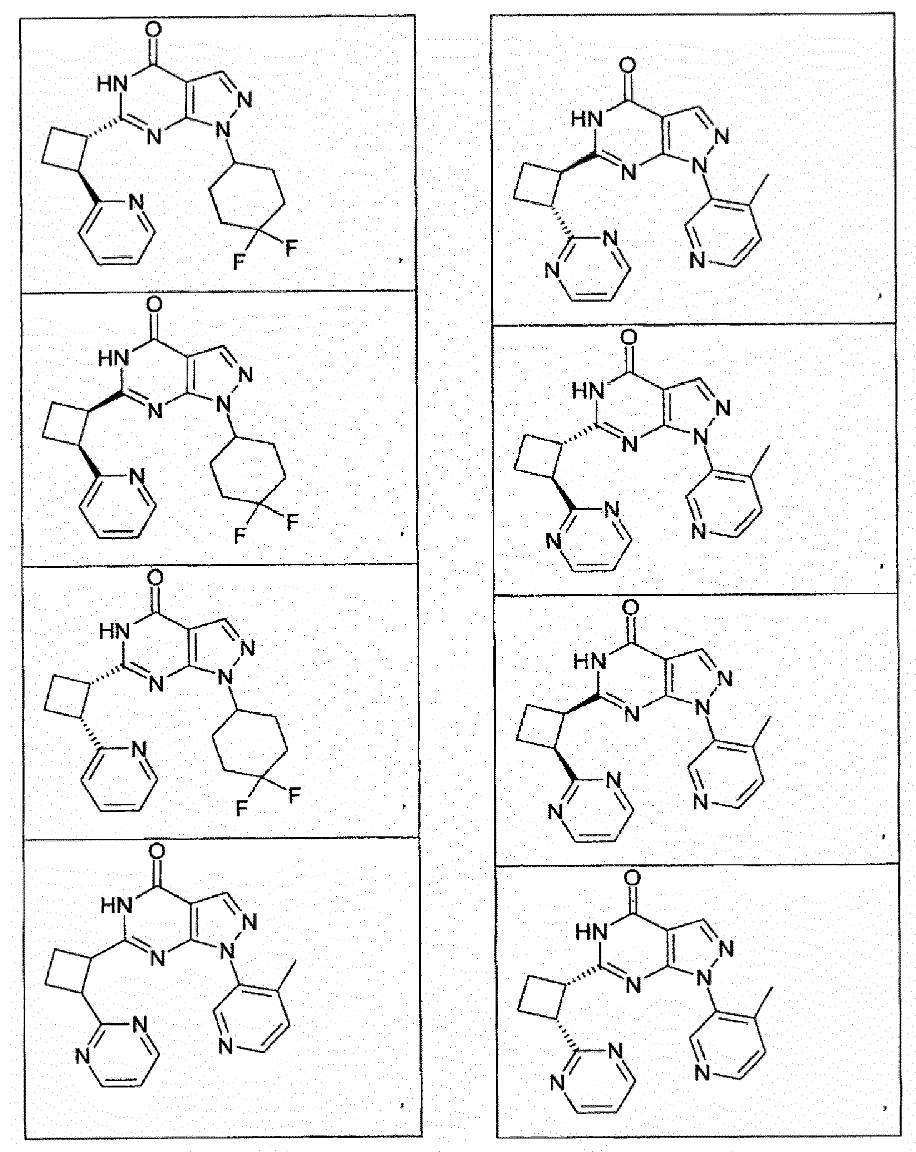 und Salze, bevorzugt pharmazeutisch akzeptable bzw. annehmbare Salze hiervon.

2. Verbindung nach Anspruch 1, wobei die Verbindung ausgewählt ist aus der Gruppe einer Verbindung gemäß der Formel (IIa) 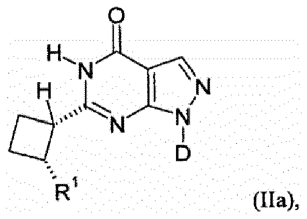 und Salze, bevorzugt pharmazeutisch akzeptable bzw. annehmbare Salze hiervon.

3. Verbindung nach Anspruch 1, wobei die Verbindung ausgewählt ist aus der Gruppe einer Verbindung gemäß der Formel (IIb) 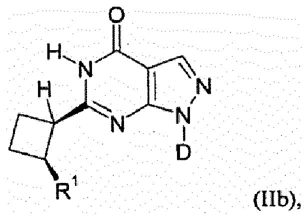 und Salze, bevorzugt pharmazeutisch akzeptable bzw. annehmbare Salze hiervon.

4. Verbindung nach Anspruch 1, wobei die Verbindung ausgewählt ist aus der Gruppe einer Verbindung gemäß der Formel (IIc) 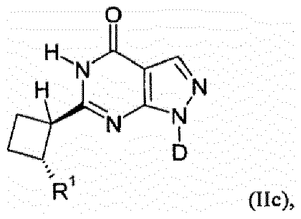 und Salze, bevorzugt pharmazeutisch akzeptable bzw. annehmbare Salze hiervon.

5. Verbindung nach Anspruch 1, wobei die Verbindung ausgewählt ist aus der Gruppe einer Verbindung gemäß der Formel (IId) 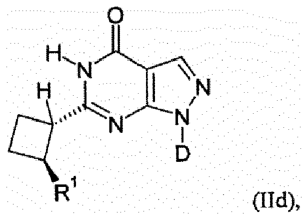 und Salze, bevorzugt pharmazeutisch akzeptable bzw. annehmbare Salze hiervon.

6. Verbindung nach irgendeinem der Ansprüche 1 bis 5 zur Verwendung als ein Medikament oder zur Verwendung als pharmazeutisch wirksamer Bestandteil in einem Medikament, bevorzugt einem Medikament zur Verwendung in einem therapeutischen oder prophylaktischen Verfahren, bevorzugt in einem therapeutischen Verfahren.

7. Verbindung nach irgendeinem der Ansprüche 1 bis 5 zur Verwendung in
(a) der Behandlung einer Erkrankung des ZNS (Zentralen Nervensystems), bevorzugter einer ZNS-Erkrankung, deren Behandlung durch die Inhibierung von PDE9 zugänglich ist,
(b) der Behandlung einer Erkrankung, die durch die Inhibierung von PDE9 zugänglich ist,
(c) der Behandlung oder der Verbesserung bzw. Linderung oder der Vorbeugung, bevorzugt der Behandlung eines Zustands, ausgewählt aus der Gruppe, bestehend aus Beeinträchtigungen der kognitiven Fähigkeiten in Zusammenhang mit einer Krankheit oder einem Zustand, ausgewählt aus der Gruppe: Auffassung, Konzentration, Wahrnehmung, Lernfähigkeit oder Gedächtnis, wobei bevorzugt die Behandlung, Linderung oder Vorbeugung der Beeinträchtigung der kognitiven Fähigkeiten in Zusammenhang steht mit altersbedingten Beeinträchtigungen der Lernfähigkeit und des Gedächtnisses, altersbedingtem Gedächtnisverlust, vaskulärer Demenz, kraniozerebralem Trauma, Schlaganfall, Demenz nach Schlaganfall (Post-Schlaganfall-Demenz), post-traumatischer Demenz, allgemeinen Beeinträchtigungen der Konzentrationsfähigkeit, Beeinträchtigungen der Konzentrationsfähigkeit bei Kindern mit Lern- und Gedächtnisproblemen, Alzheimer Erkrankung, Lewy-Body-Demenz, Demenz mit Degeneration der Frontallappen, einschließlich Pick-Syndrom, Parkinson Erkrankung, progressiver Kernlähmung, Demenz mit kortikobasaler Degeneration, amyotropher Lateralsklerose (ALS), Huntington Erkrankung, Multipler Sklerose, Thalamus-Degeneration, Kreutzfeld-Jacob-Demenz, HIV-Demenz, Epilepsie, Temporallappenepilepsie, Schizophrenie, Schizophrenie (mit Demenz), Korsakoff-Psychose oder Beeinträchtigungen der kognitiven Fähigkeiten in Zusammenhang mit Depression oder bipolarer Störung,
(d) der Behandlung der Alzheimer Erkrankung oder mit Alzheimer Erkrankung in Zusammenhang stehenden Beeinträchtigungen der kognitiven Fähigkeiten,
(e) der Behandlung von Schizophrenie oder mit Schizophrenie in Zusammenhang stehenden Beeinträchtigungen der kognitiven Fähigkeiten,
(f) der Behandlung von Epilepsie oder mit Epilepsie in Zusammenhang stehenden Beeinträchtigungen der kognitiven Fähigkeiten,
(g) der Behandlung einer Krankheit oder eines Zustands, ausgewählt aus der Gruppe, bestehend aus Schlafstörungen, bipolarer Störung, metabolischem Syndrom, Adipositas, Diabetes mellitus, Hyperglykämie, Dyslipidämie, beeinträchtigende Glucosetoleranz oder einer Erkrankung der Hoden, des Gehirns, des Dünndarms, der Skeletmuskeln, des Herzens, der Lunge, des Thymus oder der Milz.

8. Verbindung nach irgendeinem der Ansprüche 1 bis 5 zur Verwendung in der Behandlung oder Prophylaxe der Beeinträchtigung der kognitiven Fähigkeiten, bevorzugt einer Beeinträchtigung der kognitiven Fähigkeiten in Bezug auf Auffassung bzw. Wahrnehmung, Konzentration, Lernfähigkeit oder Gedächtnis.

9. Verbindung nach irgendeinem der Ansprüche 1 bis 5 zur Verwendung in der Behandlung oder Prophylaxe der Beeinträchtigung der kognitiven Fähigkeiten, bevorzugt der Beeinträchtigung der kognitiven Fähigkeiten mit Bezug auf Auffassung bzw. Wahrnehmung, Konzentration, Lernfähigkeit oder Gedächtnis, als Symptome einer anderen zugrunde liegenden Erkrankung.

10. Verbindung nach irgendeinem der Ansprüche 1 bis 5 zur Verwendung in der Verbesserung der kognitiven Fähigkeiten in Bezug auf Auffassung bzw. Wahrnehmung, Konzentration, Lernfähigkeit oder Gedächtnis.

11. Pharmazeutische Zusammensetzung, umfassend eine Verbindung nach irgendeinem der Ansprüche 1 bis 5 und einen pharmazeutischen Träger.

12. Kombination einer Verbindung nach irgendeinem der Ansprüche 1 bis 5 mit einem weiteren Wirkstoff, wobei
- die Kombination bevorzugt für die Behandlung einer Krankheit oder eines Zustands, wie in irgendeinem der Ansprüche 7 bis 10 definiert, verwendbar ist, oder
- die Kombination bevorzugt für die Verwendung in einem therapeutischen oder prophylaktischen Verfahren, bevorzugt einem therapeutischen Verfahren, für die Behandlung eines Zustands oder einer Erkrankung, wie in irgendeinem der Ansprüche 7 bis 10 definiert, ist, oder
- die Kombination für die Herstellung eines Medikaments, das bevorzugt für die Behandlung eines Zustands oder einer Erkrankung, wie in irgendeinem der Ansprüche 7 bis 10 definiert, ist.

## Revendications

1. Composé de formule (I) 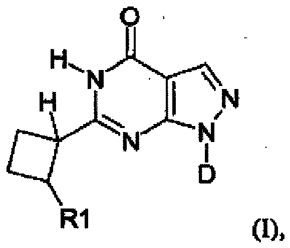 dans laquelle le composé est choisi dans le groupe de 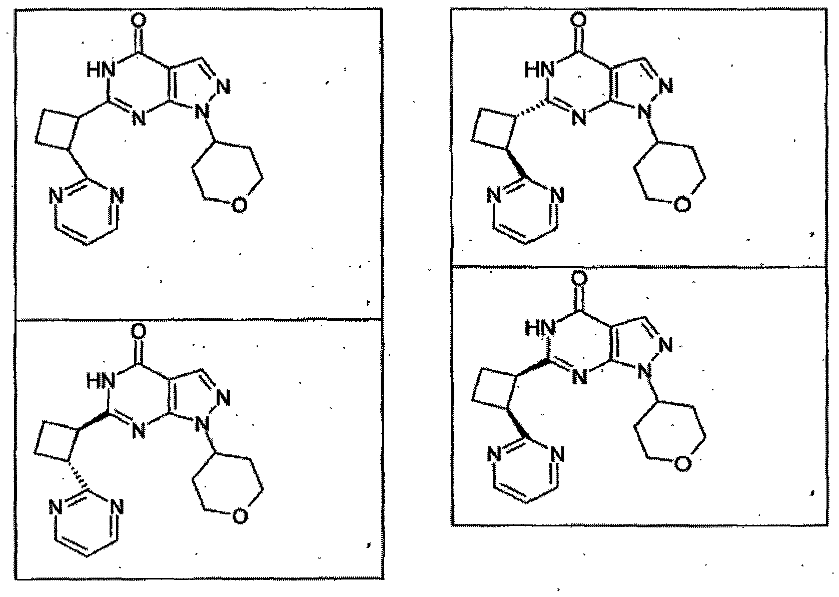 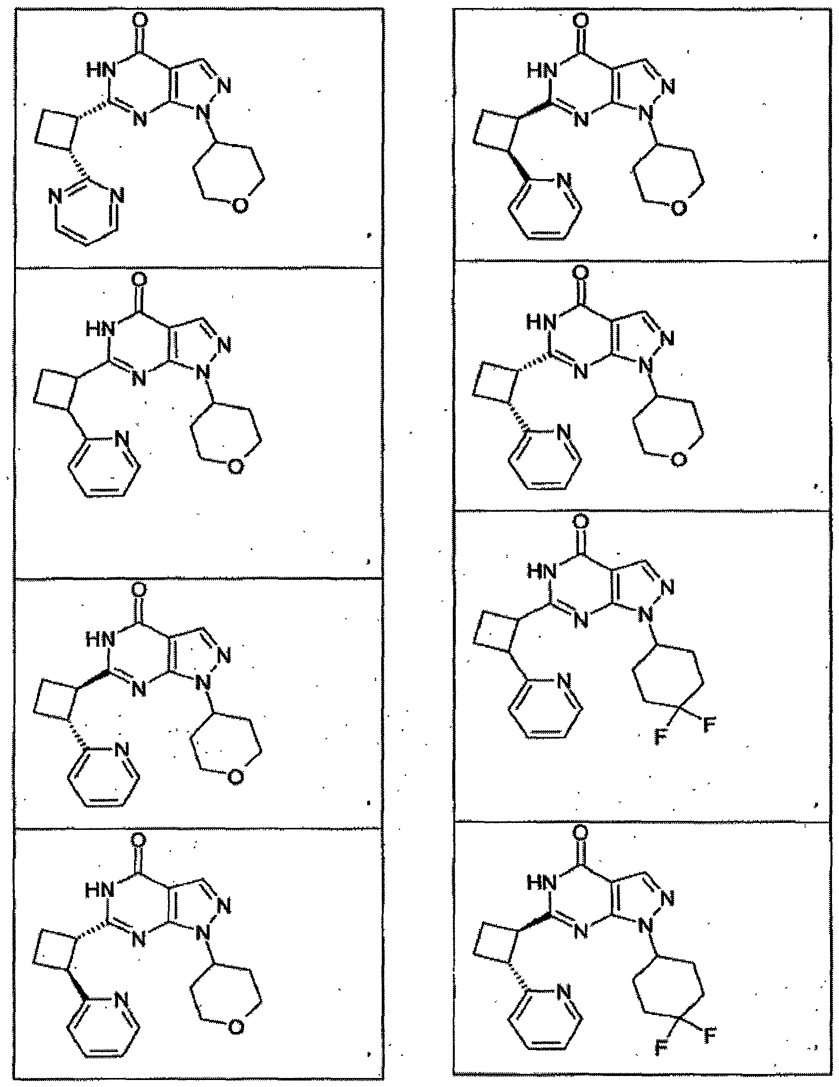 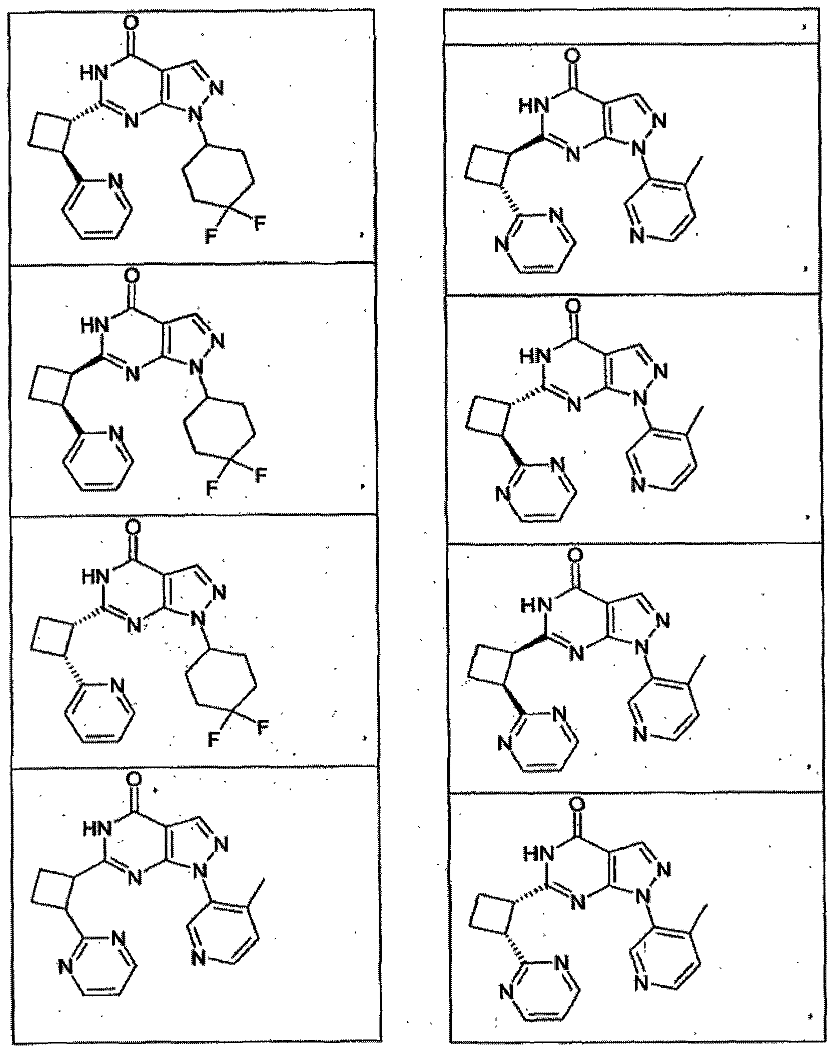 et les sels, de préférence les sels pharmaceutiquement acceptables, de ces composés.

2. Composé selon la revendication 1, dans lequel le composé est choisi dans le groupe d'un composé répondant à la formule (IIa) 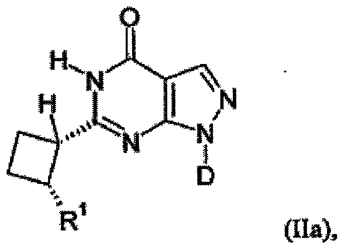 et les sels, de préférence les sels pharmaceutiquement acceptables, de ce composé.

3. Composé selon la revendication 1, dans lequel le composé est choisi dans le groupe d'un composé répondant à la formule (IIb) 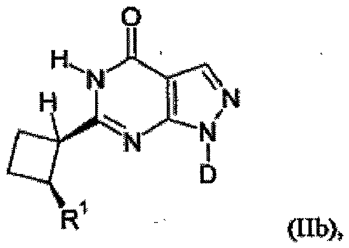 et les sels, de préférence les sels pharmaceutiquement acceptables, de ce composé.

4. Composé selon la revendication 1, dans lequel le composé est choisi dans le groupe d'un composé répondant à la formule (IIc) 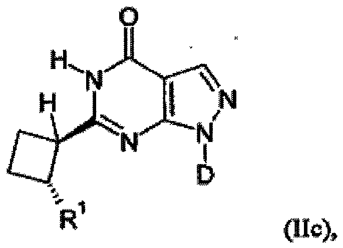 et les sels, de préférence les sels pharmaceutiquement acceptables, de ce composé.

5. Composé selon la revendication 1, dans lequel le composé est choisi dans le groupe d'un composé répondant à la formule (IId) 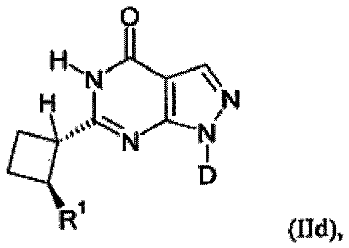 et les sels, de préférence les sels pharmaceutiquement acceptables, de ce composé.

6. Composé selon l'une quelconque des revendications 1 à 5 pour une utilisation en tant que médicament ou pour l'utilisation en tant qu'ingrédient pharmaceutiquement actif dans un médicament, de préférence un médicament pour l'utilisation dans une méthode thérapeutique ou prophylactique, de préférence dans une méthode thérapeutique.

7. Composé selon l'une quelconque des revendications 1 à 5 pour l'utilisation dans
(a) le traitement d'une maladie du SNC, de façon davantage préférée d'une maladie du SNC dont le traitement est accessible par l'inhibition de la PDE9,
(b) le traitement d'une maladie qui est accessible par l'inhibition de la PDE9,
(c) le traitement ou pour l'amélioration ou pour la prévention, de préférence pour le traitement, d'une pathologie choisie dans le groupe constitué par un déficit cognitif ayant un rapport avec une maladie ou une pathologie choisie dans le groupe des troubles de la perception, de la concentration, des processus cognitifs, de l'apprentissage ou de la mémoire, moyennant quoi de préférence le traitement, l'amélioration ou la prévention du déficit cognitif est lié à des troubles de l'apprentissage et de la mémoire associés au vieillissement, des pertes de mémoire associées au vieillissement, une démence vasculaire, un traumatisme crânien, un AVC, une démence se produisant après les AVC (démence post-AVC), une démence post-traumatique, des troubles généraux de la concentration, des troubles de la concentration chez l'enfant s'accompagnant de problèmes d'apprentissage et de mémoire, la maladie d'Alzheimer, la démence à corps de Lewy, une démence avec dégénérescence des lobes frontaux, notamment le syndrome de Pick, la maladie de Parkinson, une paralysie nucléaire progressive, une démence avec dégénérescence cortico-basale, une sclérose latérale amyotrophique (ALS), la maladie de Huntington, la sclérose en plaques, une dégénérescence thalamique, la démence de Creutzfeld-Jacob, une démence secondaire au VIH, l'épilepsie, l'épilepsie du lobe temporal, la schizophrénie, une schizophrénie (avec démence), la psychose de Korsakoff ou un déficit cognitif associé à une dépression ou à un trouble bipolaire,
(d) le traitement de la maladie d'Alzheimer ou d'un déficit cognitif associé à la maladie d'Alzheimer,
(e) le traitement de la schizophrénie ou d'un déficit cognitif associé à la schizophrénie,
(f) le traitement de l'épilepsie ou d'un déficit cognitif associé à l'épilepsie,
(g) le traitement d'une maladie ou d'une pathologie choisie dans le groupe constitué par les troubles du sommeil, le trouble bipolaire, le syndrome métabolique, l'obésité, le diabète sucré, l'hyperglycémie, la dyslipidémie, la baisse de la tolérance au glucose, ou d'une maladie des testicules, du cerveau, de l'intestin grêle, du muscle squelettique, du coeur, du poumon, du thymus ou de la rate.

8. Composé selon l'une quelconque des revendications 1 à 5 pour l'utilisation dans le traitement ou la prophylaxie d'un déficit cognitif, de préférence un déficit cognitif lié à la perception, la concentration, l'apprentissage ou la mémoire.

9. Composé selon l'une quelconque des revendications 1 à 5 pour l'utilisation dans le traitement ou la prophylaxie d'un déficit cognitif, de préférence un déficit cognitif lié à la perception, la concentration, l'apprentissage ou la mémoire, en tant que symptômes d'une autre maladie sous-jacente.

10. Composé selon l'une quelconque des revendications 1 à 5 pour l'utilisation dans l'amélioration des performances cognitives liées à la perception, la concentration, l'apprentissage ou la mémoire.

11. Composition pharmaceutique comprenant un composé selon l'une quelconque des revendications 1 à 5 et un véhicule pharmaceutique.

12. Combinaison d'un composé selon l'une quelconque des revendications 1 à 5 avec un autre agent actif, dans laquelle
- ladite combinaison est de préférence utile pour le traitement d'une maladie ou d'une pathologie telle que définie dans l'une quelconque des revendications 7 à 10 ou
- ladite combinaison est de préférence destinée à être utilisée dans une méthode thérapeutique ou prophylactique, de préférence une méthode thérapeutique, pour le traitement d'une pathologie ou d'une maladie telle que définie dans l'une quelconque des revendications 7 à 10 ou
- ladite combinaison est à utiliser dans la fabrication d'un médicament qui est de préférence destiné au traitement d'une pathologie ou d'une maladie telle que définie dans l'une quelconque des revendications 7 à 10.
